(19) Europäisches Patentamt
European Patent Office
Office européen des brevets

(11) Numéro de publication: **0 467 727 B1**

(12) # FASCICULE DE BREVET EUROPEEN

(45) Date de publication du fascicule du brevet: **13.09.95**

(51) Int. Cl.[6]: **C07C 69/88**, C07C 67/11

(21) Numéro de dépôt: **91401605.0**

(22) Date de dépôt: **17.06.91**

(54) **Procédé d'estérification des acides hydroxybenzoiques.**

(30) Priorité: **22.06.90 FR 9007818**

(43) Date de publication de la demande:
**22.01.92 Bulletin 92/04**

(45) Mention de la délivrance du brevet:
**13.09.95 Bulletin 95/37**

(84) Etats contractants désignés:
**CH DE ES FR GB IT LI NL**

(56) Documents cités:
**EP-A- 0 002 872**
**DE-B- 1 152 416**
**US-A- 3 341 575**

(73) Titulaire: **RHONE-POULENC CHIMIE**
**25, Ouai Paul Doumer**
**F-92408 Courbevoie Cédex (FR)**

(72) Inventeur: **Desmurs, Jean-Roger, La Jonquière**
**Route de Ternay,**
**Communay**
**F-69360 St Symphorien d'Ozon (FR)**
Inventeur: **Ratton, Serge**
**13, rue d'Ayen**
**F-78100 Saint Germain en Laye (FR)**

(74) Mandataire: **Dutruc-Rosset, Marie-Claude et al**
**RHONE-POULENC CHIMIE,**
**Direction de la Propriété Industrielle,**
**25, Ouai Paul Doumer**
**F-92408 Courbevoie Cédex (FR)**

**EP 0 467 727 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet un procédé d'estérification des acides hydroxybenzoïques. L'invention concerne plus particulièrement un procédé de préparation des esters de l'acide salicylique et de l'acide para-hydroxybenzoïque.

Un des procédés classiques d'obtention des esters des acides hydroxybenzoÏques consiste à faire réagir un acide hydroxybenzoïque avec un dérivé halogéné, notamment un halogénure d'alkyle, en milieu biphasique et en présence d'une base forte.

Un inconvénient majeur d'un tel procédé est l'existence de plusieurs réaction secondaires qui entraînent une diminution de rendement, en particulier, on constate une compétition entre la réaction d'estérification de la fonction carboxyle et la réaction de O-alkylation, de la fonction hydroxyle portée par le cycle aromatique.

Il résulte une baisse du rendement d'obtention de l'ester en raison de la formation de sous-produit.

De plus, il se produit dans les conditions de la réaction, une hydrolyse de l'halogénure mise en oeuvre, ce qui conduit à la formation d'un alcool dans la phase aqueuse qu'il est ensuite nécessaire d'éliminer. Il y a donc également perte de rendement par rapport à la quantité d'halogénure engagé.

Par ailleurs, on a décrit dans DE-B-1 152 416, un procédé de préparation d'esters d'acides carboxyliques. Ce procédé consiste à faire réagir un acide carboxylique et un halogénure d'alkyle, en présence d'une amine tertiaire. Seules, la triéthylamine et la tri-n-butylamine sont exemplifiées.

Un des objectifs de la présente invention est de fournir un procédé d'estérification des acides hydroxybenzoïques qui permet d'éviter les inconvénients précités.

Dans l'exposé qui suit de la présente invention, on entend par acide hydroxybenzoïque, tout composé aromatique ayant au moins 6 atomes de carbone portant au moins une fonction OH et une fonction COOH.

La présente invention a pour objet un procédé d'estérification d'un acide hydroxybenzoïque caractérisé par le fait qu'il consiste à faire réagit ledit acide avec un dérivé halogéné d'un hydrocarbure aliphatique, cycloaliphatique, cyclo- ou arylaliphatique, en phase liquide essentiellement homogène, en présence d'une amine tertiaire non quaternisable.

On utilisera ci-après le terme générique de "dérivé halogéné" pour désigner les composés halogénés précités.

Par l'expression "amine non quaternisable", on entend une amine qui, placée dans les conditions de la réaction mais en l'absence du substrat "acide hydroxybenzoïque" n'est pas quaternisée par le dérivé halogéné ou ne l'est qu'à un faible taux correspondant à un taux de transformation du dérivé halogéné en amine quaternisée au plus égal à 10 % et, de préférence, compris entre 0 et 5 %

Conformément au procédé de l'invention, on fait réagir l'acide hydroxybenzoïque et le dérivé halogéné en phase liquide essentiellement homogène. On entend par cette expression que les différents réactifs sont liquides dans les conditions réactionnelles mais le procédé n'exclut pas la présence d'éventuels catalyseurs sous forme solide.

La réaction d'estérification selon l'invention peut être conduite en l'absence de solvant ou en présence de solvant organique. D'une manière préférentielle, on préfère choisir les conditions qui permettent de travailler en milieu homogène.

Le procédé de l'invention s'applique à tout acide hydroxybenzoïque et tout particulièrement à ceux répondant à la formule (I) suivante :

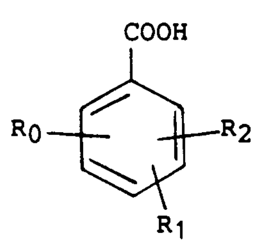

(I)

dans ladite formule (I)

- $R_O$ représente un groupe hydroxyle
- $R_1$ et $R_2$ identiques ou différents, symbolisent :
  . un atome d'hydrogène,
  . un groupe -OH,

. un radical alkyle linéaire ou ramifié ayant de 1 à 6 atomes de carbone,
. un radical alkoxy du type $R_3$-O-, où $R_3$ représente un radical alkyle linéaire ou ramifié ayant de 1 à 6 atomes de carbone,
. un groupe -COOH
. un groupe -CHO
. un groupe acyle ayant de 2 à 6 atomes de carbone
. un groupe $-COOR'_3$, $R_{3'}$ représentant un radical alkyle linéaire ou ramifié ayant de 1 à 4 atomes de carbone,
. un groupe $-NO_2$,
. un atome d'halogène de préférence de fluor, de chlore, de brome,
. un groupe $-CF_3$.

Parmi les composés de formule (I), on met en oeuvre plus particulièrement ceux répondant à la formule (I) dans laquelle $R_1$ et $R_2$ identiques ou différents représentent un atome d'hydrogène, un groupe hydroxyle, un radical alkyle ou alkoxy linéaire ou ramifié ayant de 1 à 6 atomes de carbone, de préférence de 1 à 4 atomes de carbone.

Encore plus préférentiellement, on choisit les composés de formule (I) dans laquelle $R_1$ et $R_2$ identiques ou différents sont un atome d'hydrogène, un groupe hydroxyle, un radical méthyle, un radical méthoxy.

A titre illustratif de composés répondant à la formule (I), on peut mentionner :

- acide hydroxy-2 benzoïque (acide salicylique)
- acide hydroxy-3 benzoïque
- acide hydroxy-4 benzoïque
- acide méthyl-3 salicylique
- acide méthyl-4 salicylique
- acide méthyl-5 salicylique
- acide hydroxy-3 méthyl-4 benzoïque
- acide méthoxy-3 salicylique
- acide méthoxy-4 salicylique
- acide méthoxy-5 salicylique
- acide hydroxy-3 méthoxy-4 benzoïque (acide isovanillique)
- acide hydroxy-4 méthoxy-3 benzoïque (acide vanillique)
- acide hydroxy-3 diméthoxy-4,5 benzoïque
- acide hydroxylé diméthoxy-3,5 benzoïque (acide syringique)
- acide hydroxy-5 isophtalique
- acide amino-3 salicylique
- acide amino-4 salicylique
- acide amino-5 salicylique
- acide hydroxy-3 amino-2 benzoïque
- acide nitro-3 salicylique
- acide hydroxy-3 nitro-4 benzoïque
- acide hydroxy-4 nitro-3 benzoïque
- acide hydroxy-3 méthyl-4 nitro-2 benzoïque
- acide diiodo-3,5 salicylique
- acide dihydroxy-2,3 benzoïque
- acide dihydroxy-2,4 benzoïque
- acide dihydroxy-2,5 benzoïque
- acide dihydroxy-2,6 benzoïque
- acide dihydroxy-3,4 benzoïque (acide protocatéchique)
- acide dihydroxy-3,5 benzoïque
- acide dihydroxy-3,5 méthyl-4 benzoïque
- acide trihydroxy-2,3,4 benzoïque
- acide trihydroxy-2,4,6 benzoïque
- acide trihydroxy-3,4,5 benzoïque

Les acides hydroxybenzoïques choisis préférentiellement sont l'acide salicylique et l'acide hydroxy-4 benzoïque.

Le procédé de l'invention consiste donc à mettre en contact au moins un des acides hydroxybenzoïques précités avec au moins un dérivé halogéné que l'on peut représenter symboliquement par la formule (II) :

$R_4$-X (II)

dans ladite formule (II) :

- X est un atome d'halogène : ledit atome d'halogène ne devant pas être en position vinylique ou alcynyle
- $R_4$, éventuellement substitué, représente un radical aliphatique acyclique, saturé ou insaturé, linéaire ou ramifié ; un radical cycloaliphatique, saturé ou insaturé, monocylique ou polycyclique ; un radical cyclo- ou arylaliphatique saturé ou insaturé, linéaire ou ramifié.

L'atome d'halogène peut être le chlore, le brome ou l'iode mais on choisit, de préférence, les deux premiers.

Le radical $R_4$ représente plus particulièrement :

1° - un radical alkyle, alcényle, alcadiényle, alcynyle, linéaire ou ramifié ayant, de préférence, de 1 à 12 atomes de carbone ; la chaîne hydrocarbonée pouvant être éventuellement :

- interrompue par un des groupes suivants :

  -O-, -CO-, -COO-,

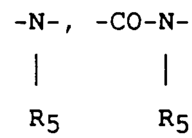

  dans ces formules, $R_5$ représente un atome d'hydrogène ou un radical alkyle ayant de 1 à 4 atomes de carbone, un radical cyclohexyle ou phényle, $R_5$ pouvant représenter un atome d'hydrogène dans la mesure où l'atome d'azote ne se quaternise pas,

- et/ou porteuse de l'un des substituants Y suivants :

  un groupe -OH, un groupe -COOH, un groupe -CHO, un groupe $-NO_2$, un groupe -C≡N, un groupe amine non quaternisable ou N- protégé, un atome d'halogène de préférence, chlore ou brome, un groupe $-CF_3$.

A titre d'exemples de radicaux aliphatiques $R_4$, on peut citer les radicaux méthyle, éthyle, propyle, isopropyle, butyle, isobutyle, sec-butyle, tert-butyle, éthyl-2 butyle, pentyle, isopentyle, néopentyle, tert-pentyle, méthyl-2 pentyle, hexyle, éthyl-2 hexyle ; allyle, méthyl-3 butène-2 yle, hexène-3 yle ; méthoxyméthyle, acétamidométhyle.

2° - un radical cycloalkyle ou cycloalcényle ayant de préférence de 5 à 7 atomes de carbone éventuellement porteur d'un ou plusieurs substituants Y tels que précités et/ou d'un ou plusieurs substituants Z qui peuvent être : un radical alkyle linéaire ou ramifié ayant de 1 à 4 atomes de carbone, un radical alkoxy linéaire ou ramifié ayant de 1 à 4 atomes de carbone, un radical de formule :

$R_5$-CO-, $R_5$-COO-, $R_5$-O-CO-,

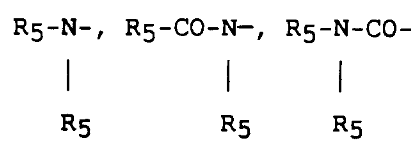

dans lesdites formules, $R_5$ ayant la signification donnée précédemment ; lesdits radicaux pouvant être polycycliques de type "pontés".

Comme exemples de radicaux cycloaliphatiques $R_4$, on peut mentionner le radical cyclohexyle et le radical cyclohexène-1 yle-1.

3° - un radical aliphatique acyclique, saturé ou insaturé, linéaire ou ramifié, tel que précisé sous 1°, porteur d'un substituant cyclique. Par cycle, on entend un cycle carbocyclique ou hétérocyclique, saturé, insaturé ou aromatique.

Le radical aliphatique acyclique peut être relié au cycle par un lien valentiel ou par l'un des groupes suivants :

-O-, -CO-, -COO-,

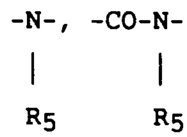

dans lesdites formules, $R_5$ ayant la signification donnée précédemment.

Comme exemples de cycles, on peut envisager :

a) un radical cycloaliphatique saturé ou insaturé, monocyclique ou polycyclique tel que mentionné sous 2°.

b) un radical phényle, tolyle, xylyle, éventuellement porteur d'un ou plusieurs substituants Y ou Z.

c) un radical hétérocyclique monocyclique, saturé, insaturé ou aromatique et comportant en tant qu'hétéroatomes un ou plusieurs atomes d'oxygène, d'azote ou de soufre, contenant 4 à 6 atomes dans le cycle : ledit radical pouvant être porteur d'un ou plusieurs substituants Y ou Z.

A titre illustratif de radicaux hétérocycliques, on peut citer les radicaux pyrrolidinyle, imidazolidinyle, pipéridyle, furyle, pyrrolyle, thiényle, isoxazolyle, furazannyle, isothiazolyle, imidazolyle, pyrazolyle, pyridyle, pyridazinyle, pyrimidinyle.

d) un radical constitué par un enchaînement de 2 à 4 groupes tels que définis aux paragraphes a et/ou b et/ou c, liés entre eux par un lien valentiel et/ou par un des groupes suivants : -O-, -S-,

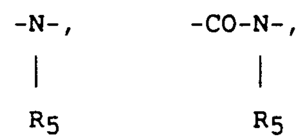

-CO-, -COO-, $-SO_2-$,

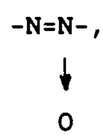

-N = N-,

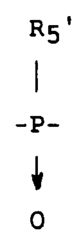

et/ou encore au moins un groupe de type alcoylène ou alcoylidène ayant de 1 à 4 atomes de carbone ; dans lesdites formules $R_5$ et $R_{5'}$ représentent un atome d'hydrogène, un radical alkyle ayant de 1 à 4 atomes de carbone, un radical cyclohexyle ou phényle et $R_5$ pouvant représenter un atome d'hydrogène dans la mesure où l'atome d'azote ne se quaternise pas.

On peut citer plus particulièrement les radicaux biphényle, méthylène-1,1′ biphényle, oxy-1,1′ biphényle, (carboxy-4 phénoxy-4) phényle, méthylène dioxy-3,4 phényle, acétyl-2 phényle, acétyl-4 phényle, benzoyl-2 phényle, benzoyl-4 phényle, benzoyloxy-3 éthyl-2 phényle, acétoxy-2 phényle, acétoxy-4 phényle, acétamido-2 phényle, acétamido-4 phényle.

e) un radical aromatique constitué par un ensemble de 2 à 3 cycles aromatiques carbocycliques et/ou hétérocycliques et formant entre eux des systèmes orthocondensés.

Les cycles aromatiques sont de préférence des noyaux benzéniques et pyridiniques.

On donne ci-après, à titre illustratif des exemples des radicaux aromatiques polycycliques : les radicaux anthryle, quinolyle, naphtyridinyle, benzofurannyle, indolyle.

Comme exemples de radicaux cyclo- ou arylaliphatiques $R_4$ porteurs d'un substituant cyclique, on peut citer préférentiellement les radicaux cyclohexylméthyle, cyclohexylbutyle, benzyle, phényl-2 éthyle, [(butyl-2)-4 phényl]-2 éthyle, styryle, $\alpha$-phénylcyclohexylméthyle, phénoxyméthyle, phénoxyéthyle.

Dans la définition du radical $R_4$ du dérivé halogéné de formule (II), il est noté que celui-ci peut porter de un à plusieurs substituants Y ou Z. Par "plusieurs, on entend généralement un nombre total ne dépassant pas 3.

D'une manière préférentielle, le radical $R_4$ représente :

- un radical alkyle linéaire ou ramifié ayant de 1 à 8 atomes de carbone, tel que par exemple, le radical méthyle, éthyle, propyle, isopropyle, butyle, isobutyle, sec-butyle, tert-butyle, éthyl-2 butyle, pentyle, isopentyle, néopentyle, tert-pentyle, méthyl-2 pentyle, hexyle, éthyl-2 hexyle,
- un radical alcényle linéaire ou ramifié ayant de 2 à 8 atomes de carbone et comportant une double liaison éthylénique en $\beta$ du groupe X comme par exemple, le radical allyle, méthyl-3 butène-2 yle, hexène-3 yle,
- un radical cyclohexyle éventuellement substitué par un radical alcoyle linéaire ou ramifié ayant de 1 à 4 atomes de carbone,
- un radical arylalkyle tel que symbolisé par la formule suivante

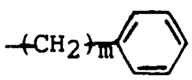

dans laquelle m est un nombre entier allant de 1 à 4, de préférence, égal à 1.

Parmi les dérivés halogénés répondant à la formule (II), on peut citer tout particulièrement, les composés suivants :

- le bromure de méthyle
- le bromure d'éthyle
- le bromure de n-propyle
- le bromoisopropane
- le bromohexane
- le bromoheptane
- le bromooctane
- le bromo-1 méthyl-2 butane
- le bromo-1 méthyl-3 butane
- le bromo-2 méthyl-2 butane
- le bromure d'allyle
- le chlorure d'allyle
- le chlorure de crotyle
- le chloro-3 méthyl-2 propène
- le chloro-1 butène-2
- le chloro-1 méthyl-3 butène-2
- le bromure de prényle
- le bromure de géranyle
- le chlorure de géranyle
- le bromocyclobutane
- le bromocyclopentane
- le bromocyclohexane
- le bromocycloheptane
- le (bromométhyl)cyclopropane
- le (chlorométhyl-2)pyridine
- le (chlorométhyl-3)pyridine
- le (chlorométhyl-4)pyridine
- le bromure de benzyle
- le chlorure de benzyle
- le chlorométhyl-4 stilbène
- le (bromométhyl-1) naphtalène

- le (bromométhyl-2) naphtalène

En ce qui concerne le choix de l'halogénure utilisé, soit chlorure ou bromure, on le détermine en fonction de la nature de l'hydrocarbure dont dérive l'halogénure et d'impératifs économiques.

On peut faire appel dans la mise en oeuvre du procédé de l'invention, à un composé bromé dans tous les cas.

Toutefois, les chlorures étant les moins chers, on cherchera à les utiliser préférentiellement, mais ceux-ci ne peuvent pas toujours être utilisés. En effet, il n'est possible selon l'invention d'avoir recours à un halogénure de type chlorure que s'il y a présence d'une insaturation sur la chaîne hydrocarbonée portant l'atome de chlore, en $\beta$ par rapport à ce dernier : l'insaturation peut être présente sur une chaîne aliphatique ou dans un cycle aromatique. Par exemple, le chlorure de benzyle peut être avantageusement utilisé.

Il a été mentionné dans la définition du radical $R_4$ que celui-ci pouvait porter un autre atome d'halogène.

Il devient ainsi possible selon le procédé de l'invention d'obtenir des diesters d'acides hydroxybenzoïques.

Il y a lieu de remarquer que les atomes d'halogène ne doivent pas être présents sur le même atome de carbone.

Des diesters d'un acide hydroxybenzoïque peuvent être obtenus dans le cas de la mise en oeuvre :

- de dérivés dibromés quels qu'ils soient, tels que par exemple, le dibromométhane, le dibromo-1,2 éthane, le dibromo-1,2 propane, le dibromo-1,3 propane, le dibromo-1,2 butane, le dibromo-1,3 butane, le dibromo-1,4 butane, le dibromo-1,6 hexane, le dibromo-1,4 butène-2, le dibromo-1,4 butyne, le bis-(bromométhyl)-1,4 benzène,
- de dérivés dichlorés présentant une insaturation en position $\beta$ par rapport aux atomes de chlore comme, par exemple, le dichloro-1,4 pentène-2, le bis-(chlorométhyl)-1,4 benzène,
- de dérivés mixtes bromé et chloré dans la mesure où il y a une insaturation en position $\beta$ par rapport à l'atome de chlore. Dans le cas contraire, par exemple lors de la mise en oeuvre du bromochloroéthane, il ne sera pas possible d'obtenir un diester, mais seulement un monoester $\beta$-chloré.

Selon le procédé de l'invention, il est également possible d'obtenir un diester lorsque l'un des radicaux $R_1$ ou $R_2$ de l'acide hydroxybenzoïque de formule (I) est une fonction -COOH.

Intervient également dans le procédé de l'invention, une amine tertiaire non quaternisable par le dérivé halogéné. Elle sert à neutraliser l'acide halohydrique libéré au cours de l'estérification.

Les amines utilisées comme catalyseur dans le présent procédé sont des amines tertiaires dont au moins l'un des radicaux portés par l'azote, est un radical aliphatique ramifié, et de préférence dont au moins deux des radicaux sont un radical aliphatique ramifié.

Comme exemples d'amines convenant à l'invention, on peut citer celles qui répondent la formule (III) suivante :

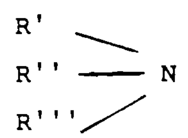
(III)

- dans laquelle R′, R″ et R‴ identiques ou différents représentent :
  . un radical aliphatique linéaire ou ramifié, saturé ou insaturé ayant de 1 à 12 atomes de carbone,
  . un radical cycloaliphatique saturé ou insaturé ayant de 5 à 7 atomes de carbone,
  . un radical phényle.
- dans ladite formule (III) :
  . au moins l'un des radicaux R′, R″ et R‴ représentant un radical aliphatique ramifié.
  . au plus l'un des radicaux R′, R″ et R‴ est un radical phényle.

A titre de radical ramifié, on choisit, de préférence, un radical aliphatique ramifié présentant une ramification sur le carbone en position $\alpha$ par rapport à l'atome d'azote.

Parmi les composés de formule (III), on choisit préférentiellement ceux répondant à la formule (III) ayant au moins deux des radicaux R′, R″ et R‴ aliphatiques ramifiés avec des ramifications situées de préférence sur le carbone en position $\alpha$ par rapport à l'atome d'azote.

A titre d'exemples de radicaux R′, R″ et R‴, on peut mentionner les radicaux méthyle, éthyle, propyle, isopropyle, butyle, isobutyle, sec-butyle, tert-butyle, pentyle, isopentyle, néopentyle, tert-pentyle, hexyle,

isohexyle, octyle, isooctyle, décyle, dodécyle, cyclohexyle, phényle.

Comme exemples d'amines tertiaires convenant bien à l'invention, on peut citer :

- la diisopropylméthylamine
- la N,N-diisopropyléthylamine
- la N,N-diisopropyl n-propylamine
- la N,N-diisopropylpropylamine
- la N,N-di sec-butyléthylamine
- la N,N,N-triisopropylamine
- la N,N-diisopropylallylamine
- la N,N-di sec-butylallylamine
- la N,N-dicyclohexyléthylamine
- le N,N-diisopropylaminoéthanol

On préfère utiliser dans le procédé de l'invention une base présentant un pka mesuré à 25 °C supérieur ou égal à 9,0 et, de préférence, supérieur ou égal à 10.

On choisit préférentiellement parmi l'ensemble des bases citées, la N,N-diisopropyléthylamine.

De plus, on préfère faire appel à une amine tertiaire non quaternisable insoluble dans l'eau car cela facilite sa récupération, en fin de réaction et évite la consommation parasite du dérivé halogéné.

Une variante préférentielle du procédé de l'invention consiste à introduire également dans le milieu réactionnel un catalyseur du type onium car il permet d'accroître la cinétique réactionnelle.

Les oniums utilisés dans le procédé de l'invention sont ceux dérivant notamment de l'azote, du phosphore, de l'arsenic, du soufre, du sélénium, de l'oxygène, du carbone ou de l'iode et coordiné à des restes hydrocarbonés. Les ions onium dérivant de l'azote, du phosphore ou de l'arsenic seront quadricoordinés, les ions onium dérivant du soufre, du sélénium, de l'oxygène, du carbone ou de S=O seront tricoordinés tandis que les ions onium dérivant de l'iode seront dicoordinés.

Les restes hydrocarbonés coordinés à ces différents éléments sont des radicaux alkyles, alcényles, aryles, cycloalkyles, aralkyles éventuellement substitués, 2 restes hydrocarbonés coordinés pouvant former ensemble un radical unique divalent.

La nature des anions liés à ces cations organiques n'a pas d'importance critique. Toutes les bases "dures" ou "intermédiaires" conviennent comme anion.

Par base "dure" ou "intermédiaire", on entend tout anion répondant à la définition classique donnée par R. PEARSON dans Journal of Chem. Ed. 45, pages 581 - 587 (1968), les termes "dure" et "intermédiaire" ayant respectivement la signification des termes de "hard" et "borderline" utilisés dans cette référence.

Parmi les ions onium pouvant être utilisés dans le présent procédé de l'invention, conviennent particulièrement ceux répondant à l'une des formules générales suivantes :

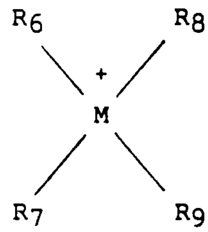

(IV)

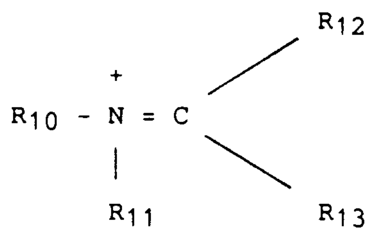

(V)

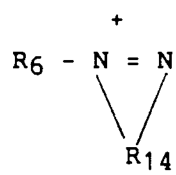

(V bis)

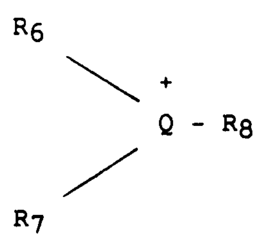

(VI)

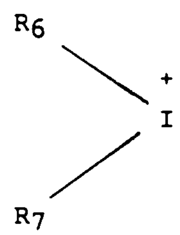

(VII)

dans lesquelles :

- M représente N, P ou As ;
- Q représente S, O, Se, S=O ou C ;
- $R_6$, $R_7$, $R_8$ et $R_9$, identiques ou différents représentent :
  . un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;
  . un radical alcényle, linéaire ou ramifié, ayant 2 à 12 atomes de carbone ;
  . un radical aryle ayant 6 à 10 atomes de carbone, éventuellement substitué par un ou plusieurs groupements ou atomes alkyle ayant 1 à 4 atomes de carbone, alkoxy, alkoxycarbonyle, le radical alkoxy ayant 1 à 4 atomes de carbone, ou halogène,

. deux desdits radicaux $R_6$ à $R_9$ pouvant former ensemble un radical alkylène, alcénylène ou alcadiènylène, linéaire ou ramifié ayant de 3 à 6 atomes de carbone ;

- $R_{10}$, $R_{11}$, $R_{12}$, $R_{13}$ sont identiques ou différents et représentent :
  . un radical alkyle linéaire ou ramifié contenant de 1 à 4 atomes de carbone ;
  . les radicaux $R_{12}$ et $R_{13}$ pouvant former ensemble un radical alkylène contenant de 3 à 6 atomes de carbone ;
  . les radicaux $R_{11}$ et $R_{12}$ ou $R_{11}$ et $R_{13}$ pouvant former ensemble un radical alkylène, alcénylène ou alcadiènylène, contenant 4 atomes de carbone et constituant avec l'atome d'azote un hétérocycle azoté ;
- $R_{14}$ représente un radical divalent formant avec les 2 atomes d'azote un cycle ayant 4 à 6 atomes pouvant comporter un ou plusieurs atomes d'azote, de soufre et/ou d'oxygène, ledit cycle pouvant être substitué par un ou plusieurs radicaux tels que $R_6$.

Parmi les bases "dures" ou "intermédiaires" pouvant constituer l'anion desdits sels d'onium, on peut citer les ions : $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, $Ph\text{-}SO_3^-$, $HSO_4^-$, $NO_3^-$, $SO_4^{2-}$, $Cl^-$, $Br^-$, $I^-$, $OH^-$, Ph représentant un radical phényle, ainsi que tous les autres anions répondant à la définition de base "dure" ou "intermédiaire" de PEARSON.

Pour des raisons de commodité de mise en oeuvre, lesdits anions pourront être choisis parmi $PO_4^{3-}$, $HPO_4^{2-}$, $H_2PO_4^-$, $CH_3SO_3^-$, $Ph\text{-}SO_3^-$, $NO_3^-$, $SO_4^{2-}$, $PF_6^-$, $Cl^-$, $Br^-$, $I^-$, Ph ayant la signification précédente. On recourra avantageusement aux anions $Br^-$ et $I^-$.

A titre d'exemples de cations organiques répondant à la formule (IV) on peut citer les cations :

- tétraméthylammonium,
- triéthylméthylammonium,
- tributylméthylammonium,
- triméthylpropylammonium,
- tétraéthylammonium,
- tétrabutylammonium,
- dodécyltriméthylammonium,
- méthyltrioctylammonium,
- heptyltributylammonium,
- tétrapropylammonium,
- tétrapentylammonium,
- tétrahexylammonium,
- tétraheptylammonium,
- tétraoctylammonium,
- tétradécylammonium,
- butyltripropylammonium,
- méthyltributylammonium,
- pentyltributylammonium,
- méthyldiétylpropylammonium,
- éthyldiméthylpropylammonium,
- tétradodécylammonium,
- tétraoctadécylammonium,
- hexadécyltriméthylammonium,
- benzyltriméthylammonium,
- benzyldiméthylpropylammonium,
- benzyldiméthyloctylammonium,
- benzyltributylammonium,
- benzyltriéthylammonium,
- phényltriméthylammonium,
- benzyldiméthyltétradécylammonium,
- benzyldiméthylhexadécylammonium,
- diméthyldiphénylammonium,
- méthyltriphénylammonium,
- butène-2-yltriéthylammonium,
- N,N-diméthyl-tétraméthylèneammonium,
- N,N-diéthyl-tétraméthylèneammonium,
- tétraméthylphosphonium,
- tétrabutylphosphonium,

- éthyltriméthylphosphonium,
- triméthylpentylphosphonium,
- octyltriméthylphosphonium,
- dodécyltriméthylphosphonium,
- triméthylphénylphosphonium,
- diéthyldiméthylphosphonium,
- dicyclohexyldiméthylphosphonium,
- diméthyldiphénylphosphonium,
- cyclohexyltriméthylphosphonium,
- triéthylméthylphosphonium,
- méthyltri(isopropyl)phosphonium,
- méthyltri(n-propyl)phosphonium,
- méthyltri(n-butyl)phosphonium,
- méthyltri(méthyl-2 propyl)phosphonium,
- méthyltricyclohexylphosphonium,
- méthyltriphénylphosphonium,
- méthyltribenzylphosphonium,
- méthyltri(méthyl-4 phényl) phosphonium,
- méthyltrixylylphosphonium,
- diéthylméthylphénylphosphonium,
- dibenzylméthylphénylphosphonium,
- éthyltriphénylphosphonium,
- tétraéthylphosphonium,
- éthyltri(n-propyl)phosphonium,
- triéthylpentylphosphonium,
- hexadécyltributylphosphonium,
- éthyltriphénylphosphonium,
- n-butyltri(n-propyl)phosphonium,
- butyltriphénylphosphonium,
- benzyltriphénylphosphonium,
- ($\beta$-phényléthyl)diméthylphénylphosphonium,
- tétraphénylphosphonium,
- triphényl(méthyl-4 phényl)phosphonium,
- tétrakis(hydroxyméthyl)phosphonium,
- tétraphénylarsonium.

Parmi les cations répondant aux formules (V) et (V bis) on peut citer les cations :

- N-méthylpyridinium,
- N-éthylpyridinium,
- N-hexadécylpyridinium,
- N-méthylpicolinium,
- triphényl-1,2,4 triazolium

A titre d'exemples de cations organiques répondant à la formule (VI), on peut citer les cations :

- triméthylsulfonium,
- triéthylsulfonium,
- triphénylsulfonium,
- triméthylsulfoxonium,
- triphénylcarbénium,
- triéthyloxonium.

A titre d'exemples de cations organiques répondant à la formule (VII), on peut citer les cations :

- diphényliodonium,
- diméthoxy-4,4' diphényliodonium (ou les composés décrits dans JACS 1958, 81, 342),
- diphényliodonium 2-carboxylate

Parmi les oniums qui peuvent être utilisés dans le cadre du présent procédé, on préférera le plus souvent les ions ammonium quaternaire, les ions phosphonium quaternaire, les ions sulfonium et les ions iodonium. Conviennent tout particulièrement bien, les ions ammonium dont les quatre groupes sont des radicaux alkyle identiques possédant de quatre à cinq atomes de carbone.

En ce qui concerne le choix du cation, on préfèrera $Br^-$ ou $I^-$.

L'onium peut être soluble dans le milieu réactionnel, on aura alors une réaction en milieu homogène.

Il peut être sous forme solide, insoluble et on aura alors une réaction en milieu biphasique solide-liquide.

Il en est ainsi notamment dans le cas où l'onium peut être supporté sur une résine minérale ou polymérique.

Comme exemples d'oniums supportés, on peut citer :

- le fluorure de tétrabutylammmonium sur gel de silice,
- le chlorure de tributylammonium sur polymère commercialisé, par exemple par la Société FLUKA,
- le chlorure de méthyltributylphosphonium lié à du polystyrène commercialisé par exemple par la Société FLUKA,
- le bromure de benzyltriméthylammonium sur polymère.

L'onium intervenant dans le procédé de l'invention peut être apporté totalement synthétisé dans le milieu réactionnel ou être préparé "in situ". Dans ce dernier cas, on peut préparer l'onium en mettant en oeuvre une quantité catalytique d'une amine tertiaire quaternisable qui en présence du dérivé halogéné de formule (II), conduit à l'ammonium désiré. Cette amine est, de préférence, la triéthylamine. Un autre mode d'obtention d'un onium consiste à générer in situ l'halohydrate de l'amine tertiaire non quaternisable par le dérivé halogéné de formule (II) en faisant réagir celle-ci avec une quantité catalytique d'un acide halohydrique qui est de préférence, l'acide bromhydrique ou l'acide chlorhydrique. On peut mettre en oeuvre ledit acide halohydrique sous forme de préférence d'une solution concentrée ou bien faire barboter celui-ci sous forme gazeuse dans le milieu réactionnel.

Conformément au procédé de l'invention, la réaction d'estérification de l'acide hydroxybenzoïque est effectuée en présence d'une amine tertiaire non quaternisable et éventuellement d'un onium, les différents réactifs étant mis en oeuvre généralement dans les proportions définies ci-après.

Le rapport molaire entre le nombre de fonctions carboxyliques de l'acide hydroxybenzoïque et le nombre d'atomes d'halogène réagissants du dérivé halogéné varie de préférence entre 0,9 et 2,0. Il est choisi préférentiellement aux environs de 1,0.

Quand l'onium est formé in situ, à partir d'une amine tertiaire quaternisable et du dérivé halogéné, on veillera à mettre en oeuvre celui-ci en une quantité excédant la quantité stoechiométrique.

En ce qui concerne la quantité d'amine tertiaire non quaternisable, celle-ci est déterminée de telle sorte qu'elle piège l'acide halohydrique formé au cours de la réaction. Elle peut donc varier de la quantité stoechiométrique jusqu'à un excès représentant environ 500 % dans le cas où ladite amine est utilisée comme solvant.

Si l'onium est formé in situ à partir de l'amine tertiaire non quaternisable et d'un acide halohydrique, il y a lieu de prévoir un excès par rapport à la stoechiométrie.

Quant à l'onium, il est mis en oeuvre en une quantité catalytique, c'est-à-dire en une quantité telle que le rapport molaire entre l'onium et l'acide hydroxybenzoïque soit situé, de préférence, entre 0,025 et 0,2.

Comme mentionné précédemment, la réaction peut être conduite en l'absence de tout solvant ou bien en présence d'un solvant organique.

Le choix du solvant est déterminé en fonction de son aptitude à solubiliser les réactifs, à savoir l'acide hydroxybenzoïque et le dérivé halogéné. De plus, il doit être inerte dans les conditions réactionnelles.

Il n'est pas nécessaire mais souhaitable que le solvant choisi solubilise l'amine tertiaire non quaternisable et l'onium.

Un mode de réalisation préférentiel de l'invention consiste à utiliser l'amine tertiaire non quaternisable comme solvant réactionnel. On choisit, de préférence, la N,N-diisopropyléthylamine.

Comme exemples de solvants réactionnels, on peut faire appel en particulier aux hydrocarbures aliphatiques ou aromatiques, aux hydrocarbures halogénés aliphatiques ou aromatiques, aux esters aliphatiques, à certains solvants aprotiques polaires, tels que les carboxamides linéaires ou cycliques comme le diméthylformamide, le diéthylformamide ou la N-méthylpyrrolidone ; les nitriles aliphatiques ou aromatiques comme l'acétonitrile, le propionitrile ou le benzonitrile.

A titre d'hydrocarbures aliphatiques ou aromatiques, on peut citer l'hexane, l'heptane, l'octane, le nonane, le décane ou le cyclohexane, le toluène, le xylène.

A titre d'hydrocarbures halogénés aliphatiques ou aromatiques, on peut mentionner : les hydrocarbures perchlorés tels que notamment le tétrachlorure de carbone, le tétrachloroéthylène, l'hexachloroéthane, l'hexachloropropène et l'hexachlorobutadiène, les hydrocarbures partiellement chlorés tels que le chlorure de méthylène, le dichloroéthane, le tétrachloroéthane, le trichloroéthylène, le chloro-1 butane, le dichloro-1,2 butane ; le monochlorobenzène, le dichloro-1,2 benzène, le dichloro-1,3 benzène, le dichloro-1,4 benzène ou des mélanges de différents chlorobenzènes ; le monobromobenzène ou des mélanges de monobromobenzène avec un ou plusieurs dibromobenzènes.

Lorsque le procédé de l'invention est mis en oeuvre en milieu solvant, la concentration de l'acide hydroxybenzoïque n'est pas critique. Elle dépendra notamment de la solubilité de celui-ci dans le solvant organique utilisé.

Selon un mode de réalisation pratique de l'invention, on peut effectuer le mélange de tous les réactifs dans un ordre quelconque.

Une réalisation préférentielle de l'invention consiste à préparer un pied de cuve comprenant l'acide hydroxybenzoïque, l'amine tertiaire non quaternisable, éventuellement l'onium et un solvant organique.

On effectue ensuite l'addition généralement progressive du dérivé halogéné dans le milieu réactionnel.

La température à laquelle est mise en oeuvre le procédé de l'invention est généralement inférieure ou égale à 120 ° C. Cette limite est imposée par des contraintes économiques car le rendement de la réaction décroît au-delà de cette température. La température limite inférieure se situe aux environs de 50 ° C. Celle-ci ne présente aucun caractère critique.

D'une manière préférentielle, la température est choisie entre 70 ° C et 120 ° C.

La pression réactionnelle n'est pas critique et est généralement la pression atmosphérique.

Pour atteindre les températures indiquées précédemment et ne pas perdre de solvant, on travaille de préférence sous pression autogène.

La durée de la réaction peut varier dans de larges limites par exemple de 15 minutes à 8 heures, mais elle est le plus souvent de 1 ou 2 heures.

En fin de réaction, l'ester de l'acide hydroxybenzoïque est séparé par les techniques classiques de séparation, telles que l'extraction par un solvant, le solubilisant par exemple, l'acétate d'éthyle, la méthylisobutylcétone, et/ou la distillation.

Si l'estérification est conduite dans un solvant organique, on dilue à l'eau le milieu réactionnel. On sépare alors la phase organique comprenant l'ester formé et le dérivé halogéné en excès et la phase aqueuse qui contient l'halohydrate de l'amine tertiaire non quaternisable, éventuellement l'onium.

On peut alors récupérer l'amine tertiaire non quaternisable en traitant la phase aqueuse avec une solution basique, de préférence la soude.

On peut ensuite la recycler.

Une autre variante de récupération de l'ester formé consiste à neutraliser par une base, de préférence, la soude, l'halohydrate de l'amine tertiaire non quaternisable.

Le sel halogéné du cation de la base de préférence NaCl ou NaBr, se trouve en phase aqueuse qui peut être séparée d'une phase organique comprenant l'ester formé, le dérivé halogéné dans le cas d'un excès et l'amine tertiaire non quaternisable.

On peut purifier l'ester par extraction à l'aide d'un solvant organique et/ou par distillation.

Il est à noter que le premier mode de traitement du milieu réactionnel est préféré, car il permet d'accéder plus directement à un ester pur.

Le procédé de l'invention convient tout-à-fait bien à la préparation des esters de l'acide salicylique, notamment : - les salicylates d'alkyle ou d'alcényle tels que par exemple, le salicylate de méthyle, le salicylate d'éthyle, le salicylate d'isopropyle, le salicylate d'amyle ou d'isoamyle, le salicylate de méthyl-2 pentyle, le salicylate de n-hexyle, le salicylate d'éthyl-2 butyle, le salicylate d'éthyl-2 hexyle, le salicylate de méthyl-2 butène-2 yle, le salicylate de cis-hexène-3 yle ; les salicylates d'alkyle ou d'alcényle porteur d'un substituant comme par exemple, le salicylate de glycol, le salicylate de monométhylamine, le salicylate de $\beta$-isopropoxyéthyle ; les salicylates de cycloalkyle, de cycloalcényle, éventuellement substitués tels que par exemple, le salicylate de cyclohexyle, le salicylate d'isopropyl-2 cyclohexyle, le salicylate de triméthyl-3,3,5 cyclohexyle, le salicylate de bornyle ; les salicylates d'arylalkyle comme, par exemple, le salicylate de benzyle.

L'invention s'applique également bien à la préparation des esters ou de l'acide o- m- ou p-aminosalicylique.

On peut mentionner notamment l'o-amino et monosalicylate de menthyle, le p-amino et monosalicylate de menthyle, le p-amino et monosalicylate d'allyle.

Le procédé de l'invention est également bien adapté à la préparation des esters de l'acide p-hydroxybenzoique. On peut citer plus particulièrement bien le p-hydroxybenzoate de méthyle, le p-hydroxybenzoate de propyle.

L'intérêt du procédé, objet de la présente invention est qu'il permet d'obtenir les esters des acides hydroxybenzoïque avec un bon taux de transformation de l'acide de départ et d'obtenir une bonne sélectivité par rapport à la O-alkylation possible dans les procédés cités de l'état de la technique.

Les exemples qui suivent illustrent l'invention sans toutefois la limiter.

Dans les exemples, les abréviations suivantes signifient :

$$TT_H = \frac{\text{nombre de moles de dérivé halogéné transformées}}{\text{nombre de moles de dérivé halogéné introduites}} \%$$

$$TT_A = \frac{\text{nombre de moles d'acide hydroxybenzoïque transformées}}{\text{nombre de moles d'acide hydroxybenzoïque introduites}} \%$$

$$RR_H = \frac{\text{nombre de moles d'ester de l'acide hydroxybenzoïque formées}}{\text{nombre de moles de dérivé halogéné introduites}} \%$$

$$RR_A = \frac{\text{nombre de moles d'ester de l'acide hydroxybenzoïque formées}}{\text{nombre de moles d'acide hydroxybenzoïque introduites}} \%$$

$$RT_H = \frac{\text{nombre de moles d'ester de l'acide hydroxybenzoïque formées}}{\text{nombre de moles de dérivé halogéné transformées}} \%$$

$$RT_A = \frac{\text{nombre de moles d'ester de l'acide hydroxybenzoïque formé}}{\text{nombre de moles d'acide hydroxybenzoïque transformées}} \%$$

Les exemples 1 à 40 illustrent la préparation de salicylate de benzyle.
Les exemples 41 à 43 concernent la préparation de salicylate d'isopropyle et de cyclohexyle.
L'exemple 44 a trait à la préparation du p-hydroxybenzoate de benzyle.

Exemple 1

Dans un réacteur en verre de 30 ml muni d'un système d'agitation, d'un dispositif de contrôle de température et d'un réfrigérant et équipé d'un système de chauffage, on charge :

- 1,1 g d'acide salicylique (8 mM)
- 1,0 g de chlorure de benzyle (8 mM)
- 1,03 g de N,N-diisopropyléthylamine (8 mM) dénommée de manière simplifiée D.I.P.E.A.

On agite le mélange réactionnel et on le porte à une température de 70 °C.
La durée totale du chauffage est de 5 heures.
En fin de réaction et après refroidissement, on ajoute 8 ml d'une solution aqueuse de soude 1 N.
On extrait les produits organiques par 3 x 5 ml d'acétate d'éthyle.
Le salicylate de benzyle est dosé par chromatographie en phase gazeuse (CPG).
Les résultats obtenus sont consignés dans le Tableau I.

Tableau I

| Référence exemple | Amine tertiaire non quaternisable | Température (°C) | $TT_H$ chlorure de benzyle | $RR_H$ salicylate de benzyle |
|---|---|---|---|---|
| 1 | D.I.P.E.A. | 70 | 84,6 | 81,9 |

Exemple 2

Dans un réacteur de 150 ml équipé comme dans l'exemple 1 avec en plus une ampoule de coulée, on charge :

- 11 g d'acide salicylique (80 mM)
- 10,3 g de N,N-diisopropyléthylamine (80 mM)

On porte le mélange réactionnel à 70° C et on introduit en 20 minutes, 10,1 g de chlorure de benzyle (80 mM).

Le mélange réactionnel est maintenu sous agitation et porté à 100° C.

La durée totale du chauffage est de 5 heures.

Après refroidissement, la masse réactionnelle est traitée comme dans l'exemple 1.

Le salicylate de benzyle est dosé par CPG.

On rassemble les résultats obtenus dans le Tableau II.

Tableau II

| Référence exemple | Amine tertiaire non quaternisable | Température (°C) | $TT_H$ chlorure de benzyle | $RR_H$ salicylate de benzyle |
|---|---|---|---|---|
| 2 | D.I.P.E.A. | 100 | 93,9 | 85,9 |

Exemples 3 à 7

Influence de la température réactionnelle

On opère comme dans l'exemple 1, en chargeant :

- 1,1 g d'acide salicylique (8 mM)
- 1,0 g de chlorure de benzyle (8 mM)
- 1,03 g de N,N-diisopropyléthylamine (8 mM)

La réaction est conduite à des températures variables, la durée du chauffage étant toujours la même soit 5 heures.

Les résultats obtenus sont consignés dans le Tableau III.

Tableau III

| Référence exemple | Amine tertiaie non quaternisable | Température (°C) | $TT_H$ chlorure de benzyle | $RR_H$ salicylate de benzyle | $RT_H$ salicylate de benzyle |
|---|---|---|---|---|---|
| 1 | D.I.P.E.A. | 70 | 84,6 | 81,9 | 96,8 |
| 3 | D.I.P.E.A. | 80 | 89,9 | 81,4 | 90,5 |
| 4 | D.I.P.E.A. | 90 | 93,8 | 85,5 | 91,1 |
| 5 | D.I.P.E.A. | 100 | 90,9 | 87,7 | 96,5 |
| 6 | D.I.P.E.A. | 110 | 92,4 | 78,2 | 84,6 |
| 7 | D.I.P.E.A. | 120 | 94,7 | 73,4 | 77,5 |

Il ressort de l'examen du Tableau que la température optimale correspondant à la meilleure sélectivité de la réaction se situe vers 100° C et qu'au-delà de cette température, on observe une baisse de sélectivité en ester formé.

Exemples 8 à 13

Influence de la durée réactionnelle

On opère comme dans l'exemple 1, en chargeant :

- 1,1 g d'acide salicylique (8 mM)
- 1,0 g de chlorure de benzyle (8 mM)
- 1,03 g de N,N-diisopropyléthylamine (8 mM)

La réaction est effectuée à 100° C, pendant des durées variables mentionnées dans le tableau récapitulatif suivant :

Tableau IV

| Référence exemple | Durée (h) | Température (°C) | $TT_H$ chlorure de benzyle | $RR_H$ salicylate de benzyle |
|---|---|---|---|---|
| 8 | 0,5 | 100 | 84,6 | 67,0 |
| 9 | 1 | 100 | 90,9 | 67,3 |
| 10 | 2 | 100 | 93,7 | 70,7 |
| 11 | 3 | 100 | 93,3 | 67,1 |
| 12 | 4 | 100 | 92,4 | 69,6 |
| 13 | 5 | 100 | 91,3 | 68,2 |

On note que la réaction est rapide et que des durées réactionnelles prolongées sont inutiles.

Exemples 14 et 15

Influence de la quantité d'amine tertiaire non quaternisable

On opère comme dans l'exemple 1, en chargeant :

- 1,1 g d'acide salicylique (8 mM)
- 1,0 g de chlorure de benzyle (8 mM)
- x g de N,N-diisopropyléthylamine

La quantité de D.I.P.E.A. est déterminée de telle sorte que l'on ait les rapports molaires entre la D.I.P.E.A. et l'acide salicylique mentionnés dans le Tableau V suivant.

La température réactionnelle est de 70° C.

La durée totale du chauffage est 5 heures.

Tableau V

| Référence exemple | Rapport molaire D.I.P.E.A./ acide salicylique | Température (°C) | $TT_H$ chlorure de benzyle | $RR_H$ salicylate de benzyle | $RT_H$ salicylate de benzyle |
|---|---|---|---|---|---|
| 14 | 2,9 | 70 | 84,3 | 79,8 | 94,6 |
| 15 | 2,4 | 70 | 83,5 | 83,4 | 99,8 |
| 1 | 1,0 | 70 | 84,6 | 81,9 | 96,8 |

La quantité d'amine utilisée n'est pas critique dès lors qu'elle est au moins égale à la quantité stoechiométrique.

Exemples 16 à 20

Influence de la nature de l'amine tertiaire non quaternisable utilisée

On opère comme dans l'exemple 1, en chargeant :

- 1,1 g d'acide salicylique (8 mM)
- 1,0 g de chlorure de benzyle (8 mM)
- x g d'une amine tertiaire non quaternisable (24 mM)

La nature de l'amine utilisée est mentionnée dans le Tableau VI suivant.
La température réactionnelle est de 100° C.

La durée du chauffage est de 5 heures.

Tableau VI

| Référence exemple | Amine tertiaire non quaternisable | Température (°C) | $TT_H$ chlorure de benzyle | $RR_H$ salicylate de benzyle | $RT_H$ salicylate de benzyle |
|---|---|---|---|---|---|
| 16 | N,N-diisopropyl n-propylamine | 100 | 71,3 | 68,3 | 95,8 |
| 17 | N,N-diisobutyl éthylamine | 100 | 24,6 | 24,6 | 100 |
| 18 | N,N-diisopropyl allylamine | 100 | 61,7 | 50,9 | 82,5 |
| 19 | N,N-dicyclohe-xyléthylamine | 100 | 54,7 | 50,0 | 91,4 |
| 20 * | N,N-diphényl éthylamine | 100 | 4,0 | 3,5 | 87,5 |

L'examen du Tableau VI conduit aux commentaires suivants :

- la présence de radicaux isopropyle dans la structure de l'amine tertiaire est favorable.
- la présence de deux radicaux phényle dans la structure de l'amine tertiaire apparaît critique.

Exemple 21 *

On effectue un essai comparatif faisant intervenir une amine quaternisable.

On reproduit l'exemple 1, en chargeant :

- 1,1 g d'acide salicylique (8 mM)
- 1,0 g de chlorure de benzyle (8 mM)
- 2,3 g de triéthylamine (23 mM)

La température réactionnelle est de 70° C.

La durée du chauffage est de 5 heures.

Dans ces conditions, le taux de transformation du chlorure de benzyle est de 100 % mais le rendement ($RR_H$) en salicylate de benzyle n'est que de 61,1 %, ce qui correspond à un rendement (RTH) de 61,1 %.

On constate donc une baisse sensible de la sélectivité.

Exemples 22 à 28

Influence de la présence et de la nature du solvant

On conduit la réaction d'estérification de l'acide salicylique en l'absence de solvant (exemple 22) et dans une série d'essais, on fait varier la nature du solvant (exemples 23 à 28).

On opère comme dans l'exemple 1, en chargeant :

- 1,1 g d'acide salicylique (8 mM)
- 1,0 g de chlorure de benzyle (8 mM)
- 1,03 g de N,N-diisopropyléthylamine (8 mM)

- 5 ml de solvant

On agite le mélange réactionnel et on le porte à une température de 70° C.

La durée du chauffage est de 5 heures.

On rassemble les différents résultats obtenus dans le Tableau VII.

Tableau VII

| Référence exemple | Solvant | Température (°C) | $TT_H$ chlorure de benzyle | $RR_H$ salicylate de benzyle |
|---|---|---|---|---|
| 22 | - | 70 | 84,6 | 81,9 |
| 23 | heptane | 70 | 75,9 | 75,8 |
| 24 | dichloro-1,2 éthane | 70 | 34,4 | 32,0 |
| 25 | acétonitrile | 70 | 66,9 | 66,4 |
| 26 | diméthylformamide | 70 | 85,9 | 85,0 |
| 27 * | diisopropyléther | 70 | 70,1 | 21,5 |
| 28 * | éthanol | 70 | 38,5 | 27,4 |

L'utilisation de certains solvants organiques en mélange avec le N,N-diisopropyléthylamine entraîne une baisse des performances de la réaction sans doute en raison de problèmes de solubilité, de mixibilité des liquides entre eux.

Exemples 29 à 37

Influence de la présence d'un onium

On effectue la réaction d'estérification de l'acide salicylique en l'absence d'onium (exemple 29) et dans une première série d'essais, on fait varier la nature de l'onium (exemples 30 à 37).

On opère comme dans l'exemple 1, en chargeant :

- 1,1 g d'acide salicylique (8 mM)
- 1,0 g de chlorure de benzyle (8 mM)
- 1,03 g de N,N-diisopropyléthylamine (8 mM)
- x g d'un onium (0,8 mM)

La nature de l'onium utilisé est mentionnée dans le Tableau VIII.

La température réactionnelle est de 50° C.
La durée totale du chauffage est de 1 heure.

Tableau VIII

| Référence exemple | Onium | Température (°C) | $TT_H$ chlorure de benzyle | $RR_H$ salicylate de benzyle |
|---|---|---|---|---|
| 29 | - | 50 | 7,2 | 7,2 |
| 30 | $(Ph)_4\ P^+\ Cl^-$ | 50 | 28,3 | 27,9 |
| 31 | $(Ph)_4\ P^+\ I^-$ | 50 | 59,9 | 45,3 |
| 32 | $(Bu)_4\ N^+\ Br^-$ | 50 | 46,0 | 54,8 |
| 33 | $Ph\ (Me)_3\ N^+\ Br^-$ | 50 | 38,4 | 26,2 |
| 34 | $Ph\ (Me)_3\ N^+\ I^-$ | 50 | 55,2 | 50,9 |
| 35 | $(Me)_3\ S^+O\ I^-$ | 50 | 36,8 | 33,4 |
| 36 | $(Et)_4\ N^+\ I^-$ | 50 | 47,5 | 43,0 |
| 37 | $(Bu)_4\ P^+\ Cl^-$ | 50 | 32,6 | 25,0 |

Dans tous les cas, on observe une amélioration du rendement en salicylate de benzyle.

Exemple 38

Dans un réacteur tel que décrit dans l'exemple 2, on charge :

- 54,6 g d'acide salicylique (395 mM)
- 51,60 g de N,N-diisopropyléthylamine (365 mM)

On porte le mélange réactionnelle à 100° C et on introduit en 50 minutes, 46,2 g de chlorure de benzyle (365 mM).

Le mélange réactionnel est maintenu sous agitation, 70 minutes à 100° C.

Après refroidissement à 65° C, on ajoute 110 ml de méthylisobutylcétone et 100 ml d'eau.

On sépare les phases organique et aqueuse.

On soumet la phase organique à 3 lavages à l'eau et l'on dose les différents composés organiques :

- 79,3 g de salicylate de benzyle (348 mM)
- 2,4 g de chlorure de benzyle (19 mM)
- 0,2 g de N,N-diisopropyléthylamine

On détermine le rendement en salicylate de benzyle de 95,5 % et un taux de transformation du chlorure de benzyle de 94,8 %.

On traite la phase aqueuse qui renferme le chlorydrate de la N,N diisopropyléthylamine par 13 ml d'une solution de soude 10N (pH = 13). Il se forme deux phases, l'une organique contenant 47,6 g de N,N-diisopropyléthylamine soit 90 % du poids engagé et l'autre aqueuse contenant du NaCl qui est soumise à 3 extractions à la méthylisobutylcétone.

On récupère 1,57 g de N,N-diisopropyléthylamine et l'on dose 5,52 g d'acide salicylique ce qui correspond à une taux de transformation de celui-ci de 87,2 %.

On a donc récupéré l'amine à 93 %, avec une pureté de plus de 98 %.

Exemples 39 et 40

Dans ces deux exemples de préparation de salicylate de benzyle, on met en oeuvre un excès d'acide salicylique.

Dans un réacteur tel que décrit dans l'exemple 2, on charge la N,N-diisopropyléthylamine et le chlorure de benzyle et l'on introduit dans le mélange réactionnel sous agitation, l'acide salicylique en 20 minutes.

Les quantités engagées sont définies dans le tableau récapitulatif IX.

La température réactionnelle est de 55°C.

La durée totale du chauffage est de 5 heures.

A cette température, on ajoute 200 ml d'eau.

On sépare la phase organique (phase I) et la phase aqueuse.

On soumet la phase organique à un lavage à l'eau avec 2 fois x 10 ml.

L'ensemble des phases aqueuses sont réunies et lavées avec 3 x 30 ml de chlorure de méthylène (phase II).

On dose ensuite les constituants dans les phases organiques (I) et (II) par CPG.

Tableau IX

| Référence exemple | Quantité acide salicylique (g) | Quantité chlorure de benzyle (g) | Rapport molaire A.S./C.B. | Quantité D.I.P.E.A. | Température (°C) | $TT_H$ chlorure de benzyle | $RR_H$ salicylate de benzyle |
|---|---|---|---|---|---|---|---|
| 39 | 37,4 | 21,64 | 1,6 | 34,76 | 55°C | 98,7 | 99 |
| 40 | 22,85 | 15,10 | 1,4 | 21,87 | 55°C | 98,3 | 99 |

Exemples 41 et 42

Influence du dérivé halogéné

Dans les exemples suivants, on prépare différents esters de l'acide salicylique.

A cet effet, on met en oeuvre différents dérivés halogénés dont la nature est précisée dans le Tableau X.

On opère comme dans l'exemple 1, en chargeant :

- 1,1 g d'acide salicylique (8 mM)
- x g de dérivé halogéné (8 mM)

- 1,03 g de N,N-diisopropyléthylamine (8 mM)

La température réactionnelle est de 100° C.

La durée totale du chauffage est de 5 heures.

Tableau X

| Référence exemple | Dérivé halogéné | Température (°C) | $TT_A$ acide salicylique | $TT_H$ dérivé halogéné | $RR_H$ ester salicylique |
|---|---|---|---|---|---|
| 41 | bromure d'isopropyle | 100 | 55 | 51 | 50 |
| 42 | bromo cyclohexane | 100 | 58,8 | 56 | 53 |

Exemple 43

On exemplifie dans cet exemple la préparation de salicylate d'isopropyle.

Dans un réacteur tel que décrit dans l'exemple 2, on charge.:

- 22,11 g de N,N-diisopropyléthylamine (171 mM)
- 21,03 g de bromure d'isopropyle (171 mM)

Dans le mélange réactionnel maintenu sous agitation, on ajoute progressivement 23,6 g d'acide salicylique (171 mM).

Le mélange réactionnel toujours maintenu sous agitation est porté progressivement à 100°C.

La durée totale du chauffage est de 5 heures 30 minutes.

La masse réactionnelle est ensuite traitée comme dans l'exemple 39.

L'ensemble des phases aqueuses sont réunies et lavées par 2 x 50 ml de chlorure de méthylène.

On dose les différents constituants dans les phases organiques réunies, par CPG.

Dans ces conditions, les taux de transformation du bromure d'isopropyle ($TT_H$) et de l'acide salicylique ($TT_A$) sont tous deux égaux à 93 %. Le rendement en salicylate d'isopropyle ($RR_H$) est de 95,5 %.

Exemple 44

Dans cet exemple, on illustre la préparation de l'ester benzylique d'un autre acide hydroxybenzoique à savoir l'acide hydroxy-4 benzoique.

On opère comme dans l'exemple 1, en chargeant :

- 1,1 g d'acide hydroxy-4 benzoique (8 mM)
- 1,0 g de chlorure de benzyle (8 mM)
- 1,03 g de N,N-diisopropyléthylamine (8 mM)

La température réactionnelle est de 70° C.

La durée totale du chauffage est de 5 heures.

Tableau XI

| Référence exemple | Acide hydroxybenzoique | Température (°C) | $TT_H$ chlorure de benzyle | $RR_H$ p-hydroxybenzoate de benzyle |
|---|---|---|---|---|
| 44 | acide hydroxy-4 benzoique | 70 | 51,8 | 47 |

**Revendications**

1. Procédé d'estérification d'un acide hydroxybenzoïque caractérisé par le fait qu'il consiste à faire réagir ledit acide avec un dérivé halogéné d'un hydrocarbure aliphatique, cycloaliphatique, cyclo- ou arylaliphatique, en phase liquide essentiellement homogène, en présence d'une amine tertiaire non quaternisable.

2. Procédé selon la revendication 1 carctérisé par le fait que l'acide hydroxybenzoïque répond à la formule (I) suivante :

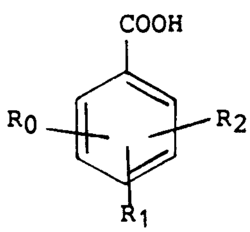

(I)

dans ladite formule (I)
- $R_0$ représente un groupe hydroxyle
- $R_1$ et $R_2$ identiques ou différents, symbolisent :
  . un atome d'hydrogène,
  . un groupe -OH,
  . un radical alkyle linéaire ou ramifié ayant de 1 à 6 atomes de carbone,
  . un radical alkoxy du type $R_3$-O-, où $R_3$ représente un radical alkyle linéaire ou ramifié ayant de 1 à 6 atomes de carbone,
  . un groupe -COOH
  . un groupe -CHO
  . un groupe acyle ayant de 2 à 6 atomes de carbone
  . un groupe $-COOR'_3$, $R_{3'}$ représentant un radical alkyle linéaire ou ramifié ayant de 1 à 4 atomes de carbone,
  . un groupe $-NO_2$,
  . un atome d'halogène de préférence de fluor, de chlore, de brome,
  . un groupe $-CF_3$.

3. Procédé selon la revendication 2 caractérisé par le fait que l'acide hydroxybenzoïque répond à la formule (I) dans laquelle $R_1$ et $R_2$ identiques ou différents représentent un atome d'hydrogène, un groupe hydroxyle, un radical alkyle ou alkoxy linéaire ou ramifié ayant de 1 à 6 atomes de carbone, de préférence de 1 à 4 atomes de carbone.

4. Procédé selon l'une des revendications 2 et 3 caractérisé par le fait que l'acide hydroxybenzoïque répond à la formule (I) dans laquelle $R_1$ et $R_2$ identiques ou différents sont un atome d'hydrogène, un groupe hydroxyle, un radical méthyle, un radical méthoxy.

5. Procédé selon l'une des revendications 2 à 4 caractérisé par le fait que l'acide hydroxybenzoïque est l'acide salicylique ou l'acide para-hydroxybenzoïque.

6. Procédé selon l'une des revendications 1 à 5 caractérisé par le fait que le dérivé halogéné répond à la formule (II) suivante :

$R_4$-X (II)

dans ladite formule (II) :

- X est un atome d'halogène : ledit atome d'halogène ne devant pas être en position vinylique ou alcynyle
- $R_4$, éventuellement substitué, représente un radical aliphatique acyclique, saturé ou insaturé, linéaire ou ramifié ; un radical cycloaliphatique, saturé ou insaturé, monocylique ou polycyclique ; un radical cyclo- ou arylaliphatique saturé ou insaturé, linéaire ou ramifié.

7. Procédé selon la revendication 6 caractérisé par le fait que le dérivé halogéné répond à la formule (II) dans laquelle le radical $R_4$ représente :

1° - un radical alkyle, alcényle, alcadiényle, alcynyle, linéaire ou ramifié ayant, de préférence de 1 à 12 atomes de carbone ; la chaîne hydrocarbonée pouvant être éventuellement :

- interrompue par un des groupes suivants :

  -O-, -CO-, -COO-,

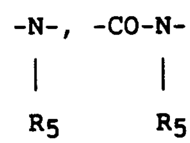

  dans ces formules, $R_5$ représente un atome d'hydrogène ou un radical alkyle ayant de 1 à 4 atomes de carbone, un radical cyclohexyle ou phényle, $R_5$ pouvant représenter un atome d'hydrogène dans la mesure où l'atome d'azote ne se quaternise pas,

- et/ou porteuse de l'un des substituants Y suivants :

  un groupe -OH, un groupe -COOH, un groupe -CHO, un groupe $-NO_2$, un groupe -C≡N, un groupe amine non quaternisable ou N-protégé, un atome d'halogène de préférence, chlore ou brome, un groupe $-CF_3$.

2° - un radical cycloalkyle ou cycloalcényle ayant de préférence de 5 à 7 atomes de carbone éventuellement porteur d'un ou plusieurs substituants Y tels que précités et/ou d'un ou plusieurs substituants Z qui peuvent être : un radical alkyle linéaire ou ramifié ayant de 1 à 4 atomes de carbone, un radical alkoxy linéaire ou ramifié ayant de 1 à 4 atomes de carbone, un radical de formule :

$R_5$-CO-, $R_5$-COO-, $R_5$-O-CO-,

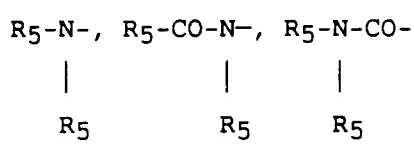

dans lesdites formules, $R_5$ ayant la signification donnée précédemment ; lesdits radicaux pouvant être polycycliques de type "pontés".

3° - un radical aliphatique acyclique, saturé ou insaturé, linéaire ou ramifié tel que précisé sous 1° porteur d'un substituant cyclique : le radical aliphatique acyclique pouvant être relié au cycle par un lien valentiel ou par l'un des groupes suivants :

-O-, -CO-, -COO-,

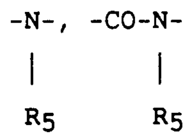

dans lesdites formules, $R_5$ ayant la signification donnée précédemment.

**8.** Procédé selon l'une des revendications 6 et 7 caractérisé par le fait que le dérivé halogéné répond à la formule (II) dans laquelle $R_4$ représente un radical aliphatique acyclique saturé ou insaturé, linéaire ou ramifié, porteur d'un substituant cyclique qui peut être :

a) un radical cycloaliphatique mono-ou polycyclique tel que défini sous 2° dans la revendication 7.

b) un radical phényle, tolyle, xylyle, éventuellement porteur d'un ou plusieurs substituants Y ou Z.

c) un radical hétérocyclique monocyclique, saturé, insaturé ou aromatique et comportant en tant qu'hétéroatomes un ou plusieurs atomes d'oxygène, d'azote ou de soufre, contenant 4 à 6 atomes dans le cycle : ledit radical pouvant être porteur d'un ou plusieurs substituants Y ou Z.

d) un radical constitué par un enchaînement de 2 à 4 groupes tels que définis aux paragraphes a et/ou b et/ou c, liés entre eux par un lien valentiel et/ou par un des groupes suivants :

-O-, -S-,

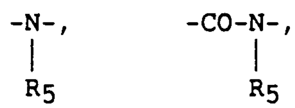

-CO-, -COO-, $-SO_2-$,

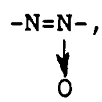

-N = N-,

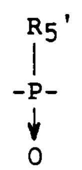

et/ou encore au moins un groupe de type alcoylène ou alcoylidène ayant de 1 à 4 atomes de carbone ; dans lesdites formules $R_5$ et $R_{5'}$ représentent un atome d'hydrogène, un radical alkyle ayant de 1 à 4 atomes de carbone, un radical cyclohexyle ou phényle et $R_5$ pouvant représenter un atome d'hydrogène dans la mesure où l'atome d'azote ne se quaternise pas.

e) un radical aromatique constitué par un ensemble de 2 à 3 cycles aromatiques carbocycliques et/ou hétérocycliques et formant entre eux des systèmes orthocondensés.

**9.** Procédé selon l'une des revendications 7 et 8 caractérisé par la fait que le dérivé halogéné répond à la formule (II) dans laquelle $R_4$ représente :

- un radical alkyle linéaire ou ramifié ayant de 1 à 8 atomes de carbone,
- un radical alcényle linéaire ou ramifié ayant de 2 à 8 atomes de carbone et comportant une double liaison éthylénique en $\beta$ du groupe X
- un radical cyclohexyle éventuellement substitué par un radical alcoyle linéaire ou ramifié ayant de 1 à 4 atomes de carbone,

- un radical arylalkyle tel que symbolisé par la formule suivante

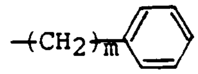

dans laquelle m est un nombre entier allant de 1 à 4.

**10.** Procédé selon la revendication 9 caractérisé par le fait que le dérivé halogéné est choisi parmi :
- le bromure d'allyle
- le chlorure d'allyle
- le bromure de benzyle
- le chlorure de benzyle
- le bromure d'isopropyle
- le chlorure de crotyle
- le chloro-1 butène-2
- le bromure de cyclohexyle

**11.** Procédé selon la revendication 1 caractérisé par le fait que l'amine tertiaire non quaternisable est une amine tertiaire dont au moins l'un des radicaux portés par l'azote, est un radical aliphatique ramifié, et de préférence dont au moins deux des radicaux sont un radical aliphatique ramifié.

**12.** Procédé selon la revendication 11 caractérisé par le fait que l'amine tertiaire non quaternisable répond à la formule (III) suivante :

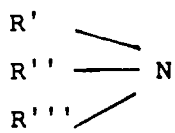 (III)

- dans laquelle R', R'' et R''' identiques ou différents représentent :
  . un radical aliphatique linéaire ou ramifié, saturé ou insaturé ayant de 1 à 12 atomes de carbone,
  . un radical cycloaliphatique saturé ou insaturé ayant de 5 à 7 atomes de carbone,
  . un radical phényle.
- dans ladite formule (III) :
  . au moins l'un des radicaux R', R'' et R''' représentant un radical aliphatique ramifié.
  . au plus l'un des radicaux R', R'' et R''' est un radical phényle.

**13.** Procédé selon l'une des revendications 11 et 12 caractérisé par le fait que l'amine tertiaire non quaternisable répond à la formule (III) dans laquelle au moins l'un des radicaux R', R'' et R''' est un radical aliphatique ramifié présentant une ramification sur le carbone en position $\alpha$ par rapport à l'atome d'azote, de préférence au moins deux des radicaux R, R'' et R''' présentent ladite ramification.

**14.** Procédé selon l'une des revendications 11 à 13 caractérisé par le fait que l'amine tertiaire non quaternisable présente un pka supérieure à 9,0, de préférence supérieur à 10.

**15.** Procédé selon l'une des revendications 11 à 14 caractérisé par le fait que l'amine tertiaire non quaternisable est choisi parmi :
- la diisopropylméthylamine
- la N,N-diisopropyléthylamine
- la N,N-diisopropyl n-propylamine
- la N,N-diisopropylpropylamine
- la N,N-di sec-butyléthylamine
- la N,N,N-triisopropylamine
- la N,N-diisopropylallylamine
- la N,N-di sec-butylallylamine

- la N,N-dicyclohexyléthylamine
- le N,N-diisopropylaminoéthanol

**16.** Procédé selon l'une des revendications 1 à 15 caractérisé par le fait que l'on opère en présence d'un onium répondant à l'une des formules générales suivantes :

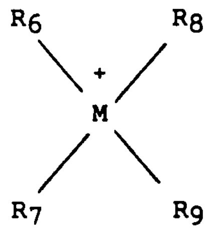

(IV)

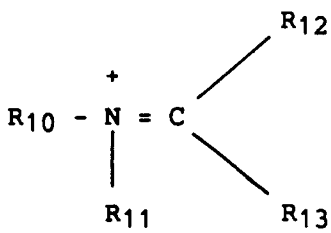

(V)

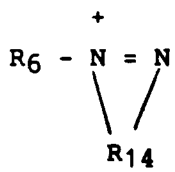

(V bis)

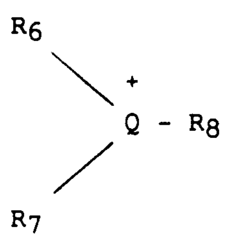

(VI)

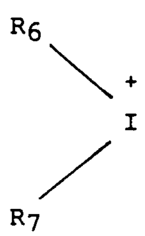

(VII)

dans lesquelles :

- M représente N, P ou As ;
- Q représente S, O, Se, S=O ou C ;

- $R_6$, $R_7$, $R_8$ et $R_9$, identiques ou différents représentent :
  . un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;
  . un radical alcényle, linéaire ou ramifié, ayant 2 à 12 atomes de carbone ;
  . un radical aryle ayant 6 à 10 atomes de carbone, éventuellement substitué par un ou plusieurs groupements ou atomes alkyle ayant 1 à 4 atomes de carbone, alkoxy, alkoxycarbonyle, le radical alkoxy ayant 1 à 4 atomes de carbone, ou halogène,
  . deux desdits radicaux $R_6$ à $R_9$ pouvant former ensemble un radical alkylène, alcénylène ou alcadiènylène, linéaire ou ramifié ayant de 3 à 6 atomes de carbone ;
- $R_{10}$, $R_{11}$, $R_{12}$, $R_{13}$, sont identiques ou différents et représentent :
  . un radical alkyle linéaire ou ramifié contenant de 1 à 4 atomes de carbone ;
  . les radicaux $R_{12}$ et $R_{13}$ pouvant former ensemble un radical alkylène contenant de 3 à 6 atomes de carbone ;
  . les radicaux $R_{11}$ et $R_{12}$ ou $R_{11}$ et $R_{13}$ pouvant former ensemble un radical alkylène, alcénylène ou alcadiènylène, contenant 4 atomes de carbone et constituant avec l'atome d'azote un hétérocycle azoté ;
- $R_{14}$ représente un radical divalent formant avec les 2 atomes d'azote un cycle ayant 4 à 6 atomes pouvant comporter un ou plusieurs atomes d'azote, de soufre et/ou d'oxygène, ledit cycle pouvant être substitué par un ou plusieurs radicaux tels que $R_6$.

**17.** Procédé selon la revendication 16 caractérisé par le fait que l'anion desdits sels d'onium est choisi parmi les ions : $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, $Ph\text{-}SO_3^-$, $HSO_4^-$, $NO_3^-$, $SO_4^{2-}$, $Cl^-$, $Br^-$, $I^-$, $OH^-$, Ph représentant un radical phényle ; l'anion desdits sels d'onium étant choisi de préférence parmi les ions $Br^-$ et $I^-$.

**18.** Procédé selon l'une des revendications 16 et 17 caractérisé par le fait que l'onium est un ion ammonium quaternaire, un ion phosphonium quaternaire, un ion sulfonium ou un ion iodonium.

**19.** Procédé selon l'une des revendications 1 à 18 caractérisé par le fait que le rapport molaire entre le nombre de fonctions carboxyliques de l'acide hydroxybenzoïque et le nombre d'atomes d'halogène réagissants du dérivé halogéné varie entre 0,9 et 2,0 et est, de préférence, égal à 1,0.

**20.** Procédé selon l'une des revendications 1 à 19 caractérisé par le fait que la quantité d'amine tertiaire non quaternisable varie de la quantité stoechiométrique jusqu'à un excès de 500 %.

**21.** Procédé selon l'une des revendications 1 à 20 caractérisé par le fait que le rapport molaire onium/acide hydroxybenzoïque est compris entre 0,025 et 0,2.

**22.** Procédé selon l'une des revendications 1 à 21 caractérisé par le fait que la réaction est conduite dans un solvant choisi parmi les hydrocarbures aliphatiques ou aromatiques, les hydrocarbures halogénés aliphatiques ou aromatiques, les solvants aprotiques polaires et les amines tertiaires non quaternisables ; le solvant choisi étant de préférence, le diméthylformamide ou la N,N-diisopropyléthylamine.

**23.** Procédé selon l'une des revendications 1 à 22 caractérisé par le fait que la température réactionnelle varie entre 70 ° C et 120 ° C.

**24.** Procédé selon l'une des revendications 1 à 23 caractérisé par le fait que l'on ajoute progressivement le dérivé halogéné dans le mélange réactionnel comprenant l'acide hydroxybenzoïque, l'amine tertiaire non quaternisable, éventuellement un onium et un solvant organique.

## Claims

**1.** A process for esterifying hydroxybenzoic acid characterised by the fact that it comprises reacting the said acid with a halogenated derivative of an aliphatic, cycloaliphatic, cyclo- or arylaliphatic hydrocarbon in essentially homogeneous liquid phase in the presence of an unquaternisable tertiary amine.

**2.** A process according to Claim 1 characterised by the fact that the hydroxybenzoic acid has the following Formula (I):

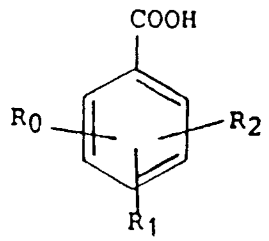

(I)

in which Formula (I)
- $R_0$ represents a hydroxyl group;
- $R_1$ and $R_2$, which are identical or different, represent: a hydrogen atom, an -OH group, a straight or branched $C_{1-6}$ alkyl radical, an alkoxy radical of the type $R_3$-O-, where $R_3$ represents a $C_{1-6}$ straight or branched alkyl radical, a -COOH group, a -CHO group, a $C_{2-6}$ acyl group, a -COOR'$_3$ group where R'$_3$ represents a straight or branched $C_{1-4}$ alkyl radical, an -$NO_2$ group, a halogen atom, preferably fluorine, chlorine or bromine, or a -$CF_3$ group.

**3.** A process according to Claim 2 characterised by the fact that the hydroxybenzoic acid has the Formula (I) in which $R_1$ and $R_2$, which are identical or different, represent a hydrogen atom, a hydroxyl group, a straight or branched $C_{1-6}$ and preferably $C_{1-4}$ alkoxy or alkyl radical.

**4.** A process according to one of Claims 2 and 3 characterised by the fact that the hydroxybenzoic acid corresponds to Formula (I) in which $R_1$ and $R_2$, which are identical or different, are hydrogen, a hydroxyl group, a methyl radical, a methoxy radical.

**5.** A process according to one of Claims 2 to 4 characterised by the fact that the hydroxybenzoic acid is salicylic acid or parahydroxybenzoic acid.

**6.** A process according to one of Claims 1 to 5 characterised by the fact that the halogenated derivative corresponds to the following Formula (II):

$R_4$-X (II)

in which Formula (II): X is a halogen atom, the said halogen atom not having to be in a vinylic or alkynylic position and $R_4$, which is optionally substituted, represents an acyclic saturated or unsaturated straight or branched aliphatic radical, a cycloaliphatic saturated or unsaturated monocyclic or polycyclic radical; or a straight or branched saturated or unsaturated cyclo- or arylaliphatic radical.

**7.** A process according to Claim 6 characterised by the fact that the halogenated derivative corresponds to Formula (II) in which the radical $R_4$ represents:

(1) a straight or branched alkyl, alkenyl, alkadienyl or alkynyl radical, preferably having 1 to 12 carbon atoms, with the hydrocarbon chain optionally interrupted by one of the following groups:
-O-, -CO-, -COO-,

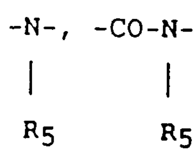

in which formulae, $R_5$ represents a hydrogen atom or a $C_{1-4}$ alkyl radical or a cyclohexyl or phenyl radical, $R_5$ representing a hydrogen atom in the case where the nitrogen atom is not quaternisable and/or carrying one of the following Y substituents: an -OH group, a -COOH group, a -CHO group, an

$-NO_2$ group, a -C≡N group, a unquaternisable or N-protected amine group a halogen atom preferably chlorine or bromine, or a $-CF_3$ group.

(2) a cycloalkyl or cycloalkenyl radical preferably having 5 to 7 carbon atoms and optionally carrying one or more Y substituents such as those cited above and/or one or more Z substituents which can be a $C_{1-4}$ straight or branched alkyl radical, a $C_{1-4}$ straight or branched alkoxy radical or a radical of formula:

$R_5$-CO-, $R_5$-COO-, $R_5$-O-CO-,

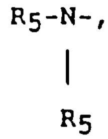, 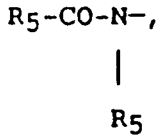, 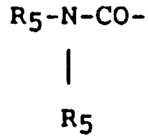

in which formulae $R_5$ is as defined above; the said radicals can be polycyclic of bridged type.

(3) a saturated or unsaturated, straight or branched acyclic aliphatic radical such as those mentioned under (1), carrying a cyclic substituent, the aliphatic acyclic radical being attached to the ring by a valency bond or by one of the following groups:

-O-, -CO-, -COO-,

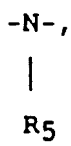, 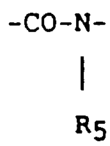

in which $R_5$ is as defined above.

**8.** A process according to one of Claims 6 and 7 characterised by the fact that the halogenated derivative has the Formula (II) in which $R_4$ represents a linear or branched, saturated or unsaturated acyclic aliphatic radical, carrying a cyclic substituent which can be:

(a) a cyclo aliphatic mono or polycyclic radical as defined in (2) in Claim 7;

(b) a phenyl, tolyl or xylyl radical, possibly carrying one or more Y or Z substituents;

(c) a saturated, unsaturated or aromatic monocyclic heterocyclic radical containing as hetero atoms one or more atoms of oxygen, nitrogen or sulphur, and containing 4 to 6 atoms in the ring, the said radical possibly carrying one or more Y or Z substituents;

(d) a radical made up of a chain of 2 to 4 groups such as those defined in paragraphs (a) and/or (b) and/or (c), linked between one another by a valency bond and/or by one of the following groups:

-O-, -S-,

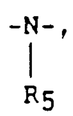, 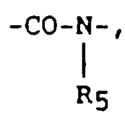,

-CO-, -COO-, $-SO_2-$,

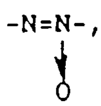,

-N = N-,

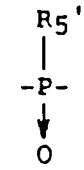

and/or again at least one $C_{1-4}$ group of the alkylene or alkylidene type; in the said formulae $R_5$ and $R_5'$ represent a hydrogen atom, a $C_{1-4}$ alkyl radical or a cyclohexyl or phenyl radical and $R_5$ can represent a hydrogen atom in the case where the nitrogen atom does not quaternize.
(e) an aromatic radical made up of an assembly of 2 or 3 aromatic carbocyclic and/or heterocyclic rings and forming between them orthocondensed systems.

**9.** A process according to Claim 8 characterised by the fact that the halogenated derivative corresponds to Formula (II) in which $R_4$ represents:
a $C_{1-8}$ straight or branched alkyl radical,
a $C_{2-8}$ straight or branched alkenyl radical containing an ethylenic double bond beta of the X group,
a cyclohexyl radical, optionally having a straight or branched $C_{1-4}$ alkyl radical substituent or
an aralkyl radical represented by the following formula

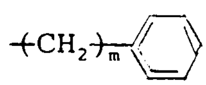

in which m is an integer from 1 to 4.

**10.** A process according to Claim 9 characterised by the fact that the halogenated derivative is chosen from allyl bromide, allyl chloride, benzyl bromide, benzyl chloride, isopropyl bromide, crotyl chloride, 1-chloro-2-butene, and cyclohexyl bromide.

**11.** A process according to Claim 1 characterised by the fact that the unquaternisable tertiary amine is a tertiary amine in which at least one of the radicals carried by the nitrogen is a branched aliphatic radical and preferably at least two such radicals are branched aliphatic radicals.

**12.** A process according to Claim 11 characterised by the fact that the tertiary unquaternisable amine corresponds to the following Formula (III):

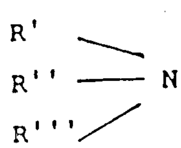

(III)

in which R', R'' and R''', which are identical or different, represent a $C_{1-12}$ straight or branched saturated or unsaturated aliphatic radical, a $C_{5-7}$ saturated or unsaturated cycloaliphatic radical, or a phenyl radical; in the said Formula (III) at least one of the radicals R', R'' and R''' representing a branched aliphatic radical, and at most one of the radicals R', R'' and R''' being a phenyl radical.

**13.** A process according to one of Claims 11 and 12 characterised by the fact that the unquaternisable tertiary amine corresponds to the Formula (III) in which at least one of the radicals R', R'' and R''' is a branched aliphatic radical having a branching on the carbon in alpha position with respect to the nitrogen atom, preferably at least two of the radicals R', R'' and R''' presenting the said branching.

**14.** A process according to one of Claims 11 to 13 characterised by the fact that the unquaternisable tertiary amine has a pKa greater than 9.0, preferably greater than 10.

**15.** A process according to one of Claims 11 to 14 characterised by the fact that the unquaternisable tertiary amine is chosen from: diisopropylmethylamine, N,N-diisopropylethylamine, N,N-diisopropyl-n-propylamine, N,N-diisopropylpropylamine, N,N-di(sec-butyl)ethylamine, N,N,N-triisopropylamine, N,N-diisopropylallylamine, N,N-di(sec-butyl)allylamine, N,N-dicyclohexylethylamine, N,N-diisopropylaminoethanol.

**16.** A process according to one of Claims 1 to 15 characterised by the fact that operation is carried out in the presence of an onium corresponding to one of the following general formulae:

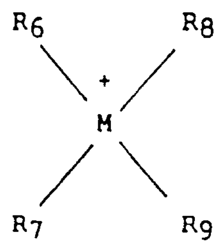

(IV)

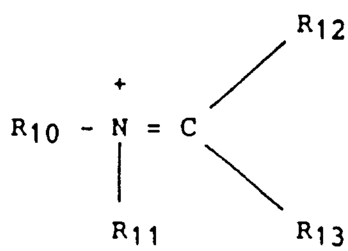

(V)

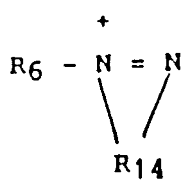

(V bis)

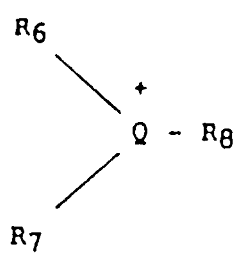

(VI)

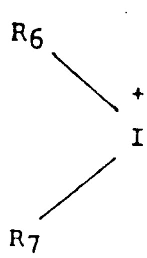

(VII)

in which:
M represents N, P or As;
Q represents S, O, Se, S=O or C;
$R_6$, $R_7$, $R_8$ and $R_9$, which are identical or different, represent a $C_{1-16}$ straight or branched alkyl radical, optionally substituted with one or more phenyl, hydroxyl, halogen, nitro, alkoxy or alkoxycarbonyl radicals, the alkoxy groups having 1 to 4 carbon atoms; a $C_{2-12}$ straight or branched alkenyl radical; a $C_{6-10}$ aryl radical possibly having one or more of the following substituent groups or atoms, namely $C_{1-4}$ alkyl, alkoxy, alkoxycarbonyl, the alkoxy radical having 1 to 4 carbon atoms, or halogen, or two of the said $R_6$ to $R_9$ radicals can together form a $C_{3-6}$ straight or branched alkylene, alkenylene or alkadienylene radical;
$R_{10}$, $R_{11}$, $R_{12}$, $R_{13}$, which are identical or different, represent a $C_{1-4}$ straight or branched alkyl radical; the radicals $R_{12}$ and $R_{13}$ can together form a $C_{3-6}$ alkylene radical; the radicals $R_{11}$ and $R_{12}$ or $R_{11}$ and $R_{13}$ can together form an alkylene, alkenylene or alkadienylene radical containing 4 carbon atoms and forming with the nitrogen atom a nitrogenous heterocycle;
$R_{14}$ represents a divalent radical forming with the 2 nitrogen atoms a ring having 4 to 6 atoms including one or more atoms of nitrogen, sulphur and/or oxygen, the said cycle optionally having one or more radicals such as $R_6$ as substituents.

**17.** A process according to Claim 16 characterised by the fact that the anion of the said onium salts is chosen from the ions: $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, $Ph\text{-}SO_3^-$, $HSO_4^-$, $NO_3^-$, $SO_4^{2-}$, $Cl^-$, $Br^-$, $I^-$, $OH^-$, where Ph represents a phenyl radical; the anion of the said onium salts being preferably chosen from $Br^-$ and $I^-$.

**18.** A process according to one of Claims 16 and 17 characterised by the fact that the onium is a quaternary ammonium ion, a quaternary phosphonium ion, a sulphonium ion or an iodonium ion.

**19.** A process according to one of Claims 1 to 18, characterised by the fact that the molar ratio between the number of carboxylic functions of hydroxybenzoic acid and the number of reacting halogen atoms of the halogenated derivative varies between 0.9 and 2.0, and is preferably equal to 1.0.

**20.** A process according to one of Claims 1 to 19 characterised by the fact that the quantity of unquaternisable tertiary amine varies from the stoichiometric quantity up to an excess of 500%.

**21.** A process according to one of Claims 1 to 20 characterised by the fact that the molar ratio onium : hydroxybenzoic acid is between 0.025 and 0.2.

**22.** A process according to one of Claims 1 to 21 characterised by the fact that the reaction is carried out in a solvent chosen from aliphatic or aromatic hydrocarbons, halogenated aliphatic or aromatic hydrocarbons, aprotic polar solvents and unquaternisable tertiary amines, the chosen solvent preferably being dimethylformamide or N,N-diisopropylethylamine.

**23.** A process according to one of Claims 1 to 22 characterised by the fact that the reaction temperature varies between 70 °C and 120 °C.

**24.** A process according to one of Claims 1 to 23 characterised by the fact that the halogenated derivative is added progressively to the reaction medium comprising the hydroxybenzoic acid, the unquaternisable tertiary amine, and possibly an onium and an organic solvent.

**Patentansprüche**

**1.** Verfahren zur Veresterung einer Hydroxybenzoesäure, dadurch gekennzeichnet, daß es darin besteht, die genannte Säure mit einem halogenierten Derivat eines aliphatischen, cycloaliphatischen, cyclo- oder arylaliphatischen Kohlenwasserstoffs in Gegenwart eines nicht quarternisierbaren tertiären Amins in flüssiger, im wesentlichen homogener Phase reagieren zu lassen.

**2.** Verfahren nach Anspruch 1, dadurch gekennzeichnet, daß die Hydroxybenzoesäure der folgenden Formel (I) entspricht:

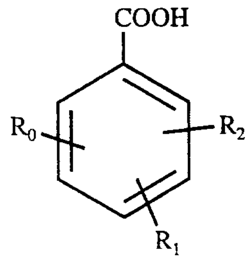

(I)

wobei in der genannten Formel (I)

- $R_0$ eine Hydroxylgruppe darstellt
- $R_1$ und $R_2$, identisch oder verschieden, folgendes symbolisieren:
  - ein Wasserstoffatom,
  - eine OH-Gruppe,
  - einen linearen oder verzweigten Alkylrest mit 1 bis 6 Kohlenstoffatomen,
  - einen Alkoxyrest des Typs $R_3$-O-, in dem $R_3$ einen linearen oder verzweigten Alkylrest mit 1 bis 6 Kohlenstoffatomen darstellt,
  - eine COOH-Gruppe,
  - eine CHO-Gruppe,
  - eine Acylgruppe mit 2 bis 6 Kohlenstoffatomen,
  - eine COOR'$_3$-Gruppe, in der R'$_3$ einen linearen oder verzweigten Alkylrest mit 1 bis 4 Kohlenstoffatomen darstellt,
  - eine $NO_2$-Gruppe,
  - ein Halogenatom, vorzugsweise Fluor, Chlor, Brom,
  - eine $CF_3$-Gruppe

**3.** Verfahren nach Anspruch 2, dadurch gekennzeichnet, daß die Hydroxybenzoesäure der Formel (I) entspricht, in der $R_1$ und $R_2$, identisch oder verschieden, ein Wasserstoffatom, eine Hydroxylgruppe, einen linearen oder verzweigten Alkoxy- oder Alkylrest mit 1 bis 6 Kohlenstoffatomen, vorzugsweise mit 1 bis 4 Kohlenstoffatomen, darstellen.

**4.** Verfahren nach einem der Ansprüche 2 und 3, dadurch gekennzeichnet, daß die Hydroxybenzoesäure der Formel (I) entspricht, in der $R_1$ und $R_2$, identisch oder verschieden, ein Wasserstoffatom, eine Hydroxylgruppe, einen Methylrest oder ein Methoxyrest sind.

**5.** Verfahren nach einem der Ansprüche 2 bis 4, dadurch gekennzeichnet, daß die Hydroxybenzoesäure Salicylsäure oder para-Hydroxybenzoesäure ist.

**6.** Verfahren nach einem der Ansprüche 1 bis 5, dadurch gekennzeichnet, daß das halogenierte Derivat der folgenden Formel (II) entspricht:

$R_4$-X (II)

in der:

- X ein Halogenatom ist: das genannte Halogenatom darf nicht in vinylischer oder alkinylischer Position sein
- R4 ein, gegebenenfalls substituierter, gesättigter oder ungesättigter, linearer oder verzweigter acyclisher aliphatischer Rest; ein gesättigter oder ungesättigter, monocyclischer oder polycyclischer cycloaliphatischer Rest; ein gesättigter oder ungesättigter, linearer oder verzweigter, cyclo- oder arylaliphatischer Rest ist.

**7.** Verfahren nach Anspruch 6, dadurch gekennzeichnet, daß das halogenierte Derivat der Formel (II) entspricht, in der der Rest R4 folgendes darstellt:

1) einen linearen oder verzweigten Alkyl-, Alkenyl-, Alkadienyl-, Alkinylrest mit vorzugsweise 1 bis 12 Kohlenstoffatomen; die Kohlenwasserstoffkette kann gegebenenfalls:

- unterbrochen werden durch eine der folgenden Gruppen:
  -O- , -CO- , -COO- ,

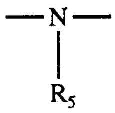 , 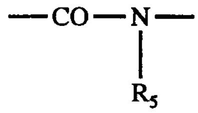

in diesen Formeln ist $R_5$ eine Wasserstoffatom oder ein Alkylrest mit 1 bis 4 Kohlenstoffatomen, ein Cyclohexyl- oder Phenylrest; $R_5$ kann ein Wasserstoffatom sein, sofern das Stickstoffatom nicht quarternisiert,
- und/oder einen der folgenden Substituenten Y tragen:
  eine OH-Gruppe, eine COOH-Gruppe, eine CHO-Gruppe, eine $NO_2$-Gruppe, eine C≡N-Gruppe, eine nicht quarternisierbare oder N-geschützte Amin-Gruppe, ein Halogenatom, vorzugsweise Chlor oder Brom, eine $CF_3$-Gruppe.

2) Ein Cycloalkyl- oder Cycloalkenylrest mit vorzugsweise 5 bis 7 Kohlenstoffatomen, der gegebenenfalls einen oder mehrere der vorgenannten Substituenten Y trägt und/oder einen oder mehrere der Substituenten Z trägt; Z kann sein: ein linearer oder verzweigter Alkylrest mit 1 bis 4 Kohlenstoffatomen, ein linearer oder verzweigter Alkoxyrest mit 1 bis 4 Kohlenstoffatomen, ein Rest der Formel:

$R_5$-CO- , $R_5$-COO- , $R_5$-O-CO- ,

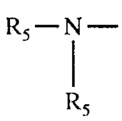 , 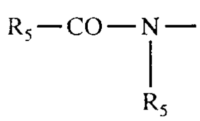 , 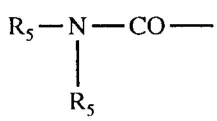

in denen $R_5$ die gleiche Bedeutung wie oben hat; und die genannten Reste polycyclisch vom "Brückentyp" sein können.

3) einen gesättigten oder ungesättigten, linearen oder verzweigten acyclischen aliphatischen Rest, der, wie unter 1) angegeben, einen cyclischen Substituenten trägt; der acyclische aliphatische Rest kann durch eine Valenzbindung oder durch eine der folgenden Gruppen zum Ring verbunden werden

-O- , -CO- , -COO- ,

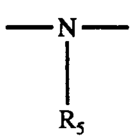 , 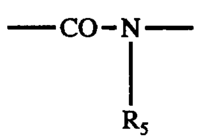

in denen $R_5$ die gleiche Bedeutung wie oben besitzt.

**8.** Verfahren nach einem der Ansprüche 6 und 7, dadurch gekennzeichnet, daß das halogenierte Derivat der Formel (II) entspricht, in der $R_4$ ein gesättigter oder ungesättigter, linearer oder verzweigter, acyclischer aliphatischer Rest und Träger eines cyclischen Substituenten ist, der folgendes sein kann:

a) ein mono- oder polycyclischer cycloaliphatischer Rest, wie er unter Punkt 2) des Anspruches 7 beschrieben wurde,

b) ein Phenyl-, Tolyl-, Xylylrest, der gegebenenfalls einen oder mehrere der Substituenten Y oder Z trägt,

c) ein gesättigter, ungesättigter oder aromatischer monocyclischer heterocyclischer Rest mit 4 bis 6 Ringatomen, der als Heteroatome ein oder mehrere Sauerstoff- Stickstoff- oder Schwefelatome trägt; dieser Rest kann einen oder mehrere der Substituenten Y oder Z tragen,

d) ein Rest, gebildet durch Kettenbildung von 2 bis 4 der unter den Abschnitten a) und/oder b) und/oder c) angegebenen Gruppen, welche untereinander durch eine Valenzbindung und/oder durch eine der folgenden Gruppen verbunden werden:
-O- , -S- ,

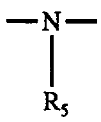 , 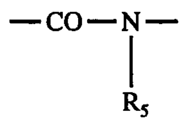 ,

-CO- , -COO- , -$SO_2$- ,

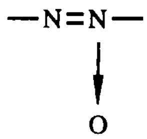 ,

-N = N- ,

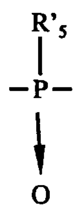

und/oder sie werden ferner durch mindestens eine Alkylen- oder Alkylidengruppe mit 1 bis 4 Kohlenstoffatomen verbunden; in den genannten Formeln sind $R_5$ und $R_5$' ein Wasserstoffatom, ein Alkylrest mit 1 bis 4 Kohlenstoffatomen, ein Cyclohexyl- oder Phenylrest, und $R_5$ kann ein Wasserstoffatom sein, sofern das Stickstoffatom nicht quarternisiert.
e) ein aromatischer Rest, gebildet aus 2 bis 3 carbocyclischen und/oder heterocyclischen aromatischen Ringen, die untereinander orthokondensierte Systeme bilden.

**9.** Verfahren nach einem der Ansprüche 7 oder 8, dadurch gekennzeichnet, daß das halogenierte Derivat der Formal (II) entspricht, in der $R_4$ folgendes darstellt:
- einen linearen oder verzweigten Alkylrest mit 1 bis 8 Kohlenstoffatomen,
- einen linearen oder verzweigten Alkenylrest mit 2 bis 8 Kohlenstoffatomen, der eine ethylenische Doppelbindung in $\beta$-Stellung in bezug auf die X-Gruppe enthält,
- einen Cyclohexylrest, der gegebenenfalls durch einen linearen oder verzweigten Alkylrest mit 1 bis 4 Kohlenstoffatomen substituiert ist,
- einen Arylalkylrest, der der folgenden Formel entspricht

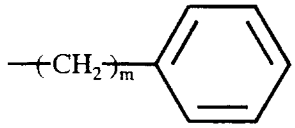

in der m eine ganze Zahl zwischen 1 und 4 ist.

**10.** Verfahren nach Anspruch 9, dadurch gekennzeichnet, daß das halogenierte Derivat ausgewählt ist, aus:
- Allylbromid
- Allylchlorid

- Benzylbromid
- Benzylchlorid
- Isopropylbromid
- Crotylchlorid
- 2-Buten-1-chlorid
- Cyclohexylbromid

**11.** Verfahren nach Anspruch 1, dadurch gekennzeichnet, daß das nicht quarternisierbare tertiäre Amin ein tertiäres Amin ist, wobei mindestens einer der am Stickstoff gebundenen Reste ein verzweigter aliphatischer Rest ist, vorzugsweise sind mindestens zwei der am Stickstoffatom gebundenen Reste ein verzweigter aliphatischer Rest.

**12.** Verfahren nach Anspruch 11, dadurch gekennzeichnet, daß das nicht quarternisierbare tertiäre Amin der folgenden Formel (III) enspricht:

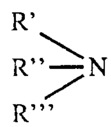

(III)

- in der R', R'' und R''', identisch oder verschieden, folgendes darstellen:
  - ein linearer oder verzweigter, gesättigter oder ungesättigter aliphatischer Rest mit 1 bis 12 Kohlenstoffatomen
  - ein gesättigter oder ungesättigter cycloaliphatischer Rest mit 5 bis 7 Kohlenstoffatomen
  - ein Phenylrest
- in der genannten Formel (III)
  - ist mindestens einer der Reste R', R'' und R''' ein verzweigter aliphatischer Rest,
  - ist höchstens einer der Reste R', R'' und R''' ein Phenylrest.

**13.** Verfahren nach Anspruch 11 oder 12, dadurch gekennzeichnet, daß das nicht quarternisierbare tertiäre Amin der Formel (III) enspricht, in der mindestens einer der Reste R', R'' und R''' ein verzweigter aliphatischer Rest ist, der eine Verzweigung an dem Kohlenstoffatom in $\alpha$-Position zum Stickstoffatom aufweist, vorzugsweise haben mindestens zwei der Reste R', R'' und R''' die genannte Verzweigung.

**14.** Verfahren nach einem der Ansprüche 11 bis 13, dadurch gekennzeichnet, daß das nicht quarternisierbare tertiäre Amin einen pkA-Wert über 9,0, vorzugsweise über 10, aufweist.

**15.** Verfahren nach einem der Ansprüche 11 bis 14, dadurch gekennzeichnet, daß das nicht quarternisierbare tertiäre Amin ausgewählt ist aus:

- Diisopropylmethylamin
- N,N-Diisopropylethylamin
- N,N-Diisopropyl-n-propylamin
- N,N-Diisopropylpropylamin
- N,N-Di-sek.-butylethylamin
- N,N-N-Triisopropylamin
- N,N-Diisopropylallylamin
- N,N-di-sek.-Butylallylamin
- N,N-Dicyclohexylethylamin
- N,N-Diisopropylaminoethanol

**16.** Verfahren nach einem der Ansprüche 1 bis 15, dadurch gekennzeichnet, daß man in Gegenwart einer Oniumverbindung verfährt, die mindestens einer der folgenden allgemeinen Formeln enspricht:

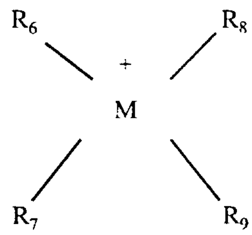

(IV)

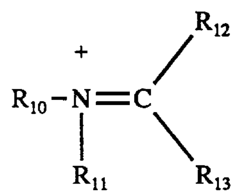

(V)

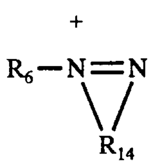

(V bis)

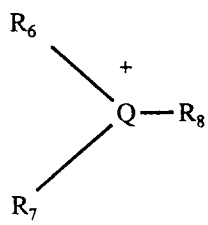

(VI)

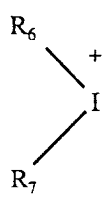

(VII)

in denen:

- M ein N-, P- oder As-Atom darstellt,
- Q ein S-, O-, Se-, C-Atom oder eine S = O-Gruppe darstellt,
- $R_6$, $R_7$, $R_8$ und $R_9$, identisch oder verschieden, folgendes darstellen:
  - einen linearen oder verzweigten Alkylrest mit 1 bis 16 Kohlenstoffatomen, der gegebenenfalls substituiert ist durch ein oder mehrere Halogenatom(e), Phenyl-, Hydroxyl-, Nitro-, Alkoxy- oder Alkoxycarbonylgruppe(n), wobei die Alkoxygruppen 1 bis 4 Kohlenstoffatome haben;
  - einen linearen oder verzweigten Alkenylrest mit 2 bis 12 Kohlenstoffatomen;
  - einen Arylrest mit 6 bis 10 Kohlenstoffatomen, der gegebenenfalls substituiert ist durch einen oder mehrere Alkylrest(e) mit 1 bis 4 Kohlenstoffatomen, Alkoxy- oder Alkoxycarbonylatome oder -reste, wobei die Alkoxyreste 1 bis 4 Kohlenstoffatome haben, oder durch Halogenatome;
  - zwei der genannten Reste $R_6$ bis $R_9$ zusammen einen linearen oder verzweigten Alkylen-, Alkenylen- oder Alkadienylenrest mit 3 bis 6 Kohlenstoffatomen bilden können;
- $R_{10}$, $R_{11}$, $R_{12}$ und $R_{13}$, identisch oder verschieden, stellen folgendes dar:

- einen linearen oder verzweigten Alkylrest mit 1 bis 4 Kohlenstoffatomen;
- die Reste $R_{12}$ und $R_{13}$ können gemeinsam einen Alkylenrest mit 3 bis 6 Kohlenstoffatomen bilden;
- die Reste $R_{11}$ und $R_{12}$ oder $R_{11}$ oder $R_{13}$ können gemeinsam einen Alkylen- Alkenylen- oder Alkadienylenrest mit 4 Kohlenstoffatomen bilden, der mit dem Stickstoffatom einen Stickstoff enthaltenden Heterocyclus bildet;

- $R_{14}$ ist ein bivalenter Rest, der mit zwei Stickstoffatomen einen Ring mit 4 bis 6 Atomen bildet, der einen oder mehrere Stickstoff-, Schwefel- und/oder Sauerstoffatom(e) tragen kann, wobei der genannte Ring durch einen oder mehrere Rest(e), wie z.B. $R_6$, substituiert sein kann.

**17.** Verfahren nach Anspruch 16, dadurch gekennzeichnet, daß das Anion der genannten Oniumsalze aus folgenden Ionen gewählt ist: $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, Ph-$SO_3^-$, $HSO_4^-$, $NO_3^-$, $SO_4^{2-}$, $Cl^-$, $Br^-$, $I^-$, $OH^-$; wobei Ph einen Phenylrest darstellt: das Anion der genannten Oniumsalze ist vorzugsweise aus den $Br^-$- und $I^-$-Ionen ausgewählt.

**18.** Verfahren nach einem der Ansprüche 16 und 17, dadurch gekennzeichnet, daß die Oniumverbindung ein quaternäres Ammoniumion, ein quaternäres Phosphoniumion, ein Sulfoniumion oder ein Iodoniumion ist.

**19.** Verfahren nach einem der Ansprüche 1 bis 18, dadurch gekennzeichnet, daß das molare Verhältnis zwischen der Anzahl der Carboxylfunktionen der Hydroxybenzoesäure und der Anzahl der reaktiven Halogenatome in dem halogenierten Derivat zwischen 0,9 und 2,0 liegt, vorzugsweise gleich 1,0 ist.

**20.** Verfahren nach einem der Ansprüche 1 bis 19, dadurch gekennzeichnet, daß die Menge an nicht quaternisierbaren tertiären Amin zwischen der stöchiometrischen Menge und einem Überschuß von 500 % liegt.

**21.** Verfahren nach einem der Ansprüche 1 bis 20, dadurch gekennzeichnet, daß das molare Verhältnis von Oniumverbindung zur Hydroxybenzoesäure zwischen 0,025 und 0,2 liegt.

**22.** Verfahren nach einem der Ansprüche 1 bis 21, dadurch gekennzeichnet, daß die Reaktion in einem Lösemittel durchgeführt wird, das aus aliphatischen oder aromatischen Wasserstoffen, aliphatischen oder aromatischen halogenierten Kohlenwasserstoffen, polaren aprotischen Lösemitteln und nicht quaternisierbaren tertiären Aminen ausgewählt ist; das Lösemittel ist vorzugsweise Dimethylformamid oder N,N-Diisopropylethylamin.

**23.** Verfahren nach einem der Ansprüche 1 bis 22, dadurch gekennzeichnet, daß die Reaktionstemperatur zwischen 70 und 120 °C liegt.

**24.** Verfahren nach einem der Ansprüche 1 bis 23, dadurch gekennzeichnet, daß man nach und nach das halogenierte Derivat zu der Reaktionsmischung hinzugibt, die die Hydroxybenzoesäure, das nicht quaternisierbare tertiäre Amin, gegebenenfalls eine Oniumverbindung und ein organisches Lösemittel enthält.